# EUROPEAN PATENT SPECIFICATION

(11) **EP 1 468 996 B1**
(45) Date of publication and mention of the grant of the patent: **26.09.2007**
(21) Application number: 04012325.9
(22) Date of filing: 13.06.2001
(51) Int. Cl.: C07D 401/14, C07D 413/14, A61K 31/538, A61P 25/18

(54) **Indole derivatives for the treatment of CNS disorders**
Indolderivate zur Behandlung von Erkrankungen des Zentralnervensystems
Derivés d'indole pour le traitement des maladies du système nerveux central

(30) Priority: 14.06.2000 DK 200000919; 16.06.2000 US 212445 P
(43) Date of publication of application: 20.10.2004
(62) Divisional of application: 01940240.3
(73) Proprietor: H. LUNDBECK A/S, 2500 Valby-Copenhagen (DK)
(72) Inventor: Bang-Andersen, Benny, 2200 Kobenhavn N (DK); Kehler, Jan, 2800 Kgs Lyngby (DK); Felding, Jacob, 2300 Kobenhavn S (DK); Andersen, Kim, 2830 Virum (DK)
(74) Representative: Nielsen, Leila

(56) References cited:
- EP-A- 1 106 605
- EP-A- 1 294 710
- WO-A-00/31074
- WO-A-01/87881
- WO-A-98/28293
- WO-A-99/11619
- WO-A-99/58525

## Description

### Field of the Invention

The present invention relates to a novel class of indole derivatives having affinity for the dopamine D₄ receptor. The compounds have antagonistic effect at the dopamine D₄ receptor and are therefore useful in the treatment of certain psychiatric and neurologic disorders, in particular psychoses. Some of the compounds also have affinity for the dopamine D₃ receptor, the 5-HT_{2A} receptor and/or the 5-HT_{2C} receptor and some of the compounds are serotonin reuptake inhibitors.

### Background of the Invention.

Dopamine D₄ ligands related to the compounds of the invention are known from WO 98/28293. The indane and dihydroindole derivatives disclosed herein have the general formula 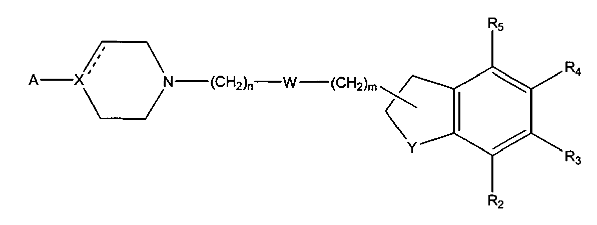 wherein A is an indole and Y is a group completing an indane, or a dihydroindole and the other substituents are as defined in the application.

WO 00/23441 discloses compounds of the general formula 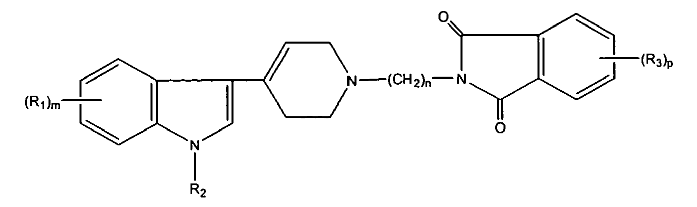 wherein the substituents R₁, R₂, R₃, m, n and p are as defined in the application. The compounds are said to show high affinity to dopamine D₂ receptors and are also said to be serotonin reuptake inhibitors. The compounds are claimed to be useful for the treatment of schizophrenia and other psychotic disorders.

Other compounds structurally related to the compounds of the invention are described in WO 99/58525. The compounds disclosed herein are said to be 5-HT_{2A} ligands and serotonin reuptake inhibitors and have the general formula 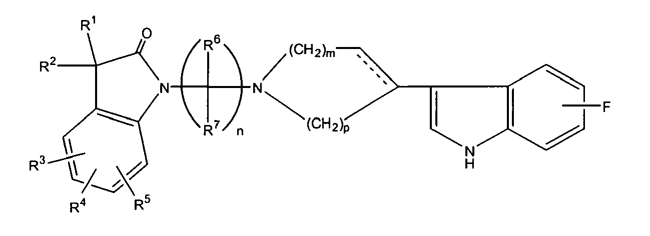 wherein the substituents are as defined in the application. The compounds are said to be useful for the treatment of schizophrenia.

WO 00/31074 relates to compounds having the formula 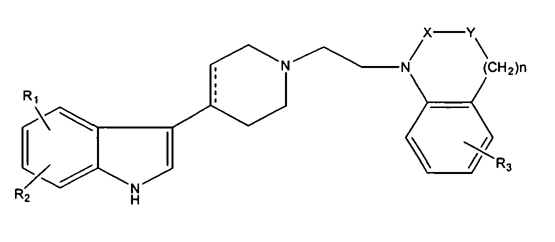 wherein X is CO or SO₂ and Y is N-R⁴ or CR⁴R⁵ and the substitutents are as described in the application. The compounds are said to be active at the 5-HT_{2A} receptor, to have 5-HT reuptake inhibiting activity and to enhance 5-HT release.

The applications, WO 94/18197, EP 329168, WO 93/16073, EP 732332, WO98/37893 and WO 95/11680, disclose dopamine D₄ ligands, which, like the compounds of the present invention, are substituted tetrahydroquinolinone and tetrahydroisoquinolinone derivatives. However, these compounds do not contain an indole as the compounds of the invention. The compounds are said to be dopamine D₄ ligands useful as antipsychotics. The compounds of WO 93/16073 are also claimed to have antagonistic activity at 5-HT₂ receptors.

Dopamine D₄ receptors belong to the dopamine D₂ subfamily of receptors, which is considered to be responsible for the antipsychotic effect of neuroleptics. The side effects of neuroleptic drugs, which primarily exert their effect via antagonism of D₂ receptors, are known to be due to D₂ receptor antagonism in the striatal regions of the brain. However, dopamine D₄ receptors are primarily located in areas of the brain other than striatum, suggesting that antagonists of the dopamine D₄ receptor will be devoid of extrapyramidal side effects. This is illustrated by the antipsychotic clozapine, which exerts higher affinity for D₄ than D₂ receptors, and is lacking extrapyramidal side effects (Van Tol et al. Nature 1991, 350, 610; Hadley Medicinal Research Reviews 1996, 16, 507-526 and Sanner Exp. Opin. Ther. Patents 1998, 8, 383-393).

A number of D₄ ligands, which were postulated to be selective D₄ receptor antagonists (L-745,879 and U-101958), have been shown to posses antipsychotic potential (Mansbach et al. Psychopharmacology 1998, 135, 194-200). However, recently it has been reported that these compounds are partial D₄ receptor agonists in *various in vitro* efficacy assays (Gazi et al. Br. J. Pharmacol. 1998, 124, 889-896 and Gazi et al. Br. J. Pharmacol. 1999, 128, 613-620). Furthermore, it was shown that clozapine, which is an effective antipsychotic, is a silent antagonists (Gazi et al. Br. J. Pharmacol. 1999, 128, 613-620).

Consequently, D₄ ligands, which are partial D₄ receptor agonists or antagonists, may have beneficial effects against psychoses.

Dopamine D₄ antagonists may also be useful for the treatment of cognitive deficits (Jentsch et al. Psychopharmacology 1999, 142, 78-84).

Further, evidence for a genetic association between the "primarily inattentive" subtype of ADHD and a tandem duplication polymorphism in the gene encoding the dopamine D₄ receptor has been published (McCracken et al. Mol. Psychiat. 2000, 5, 531-536). This clearly indicates a link between the dopamine D₄ receptor and ADHD, and ligands affecting this receptor may be useful for the treatment of this particular disorder

Dopamine D₃ receptors also belong to the dopamine D₂ subfamily of receptors, and they are preferentially located in the limbic brain regions (Sokoloff et al. Nature 1990, 347, 146-151), such as the nucleus accumbens, where dopamine receptor blockade has been associated with antipsychotic activity (Willner Int. Clinical Psychopharmacology 1997, 12, 297-308). Furthermore, an elevation of the level of D₃ receptors in the limbic part of schizophrenic brains has been reported (Gurevich et al. Arch Gen Psychiatry 1997, 54, 225-32). Therefore, D₃ receptor antagonists may offer the potential for an effective antipsychotic therapy, free of the extrapyramidal side effects of the classical antipsychotic drugs, which primarily exert their effect by blockade of D₂ receptors (Shafer et al. Psychopharmacology 1998, 135, 1-16 and Schwartz et al. Brain Research Reviews 2000, 31, 277-287).

Moreover, D₃ receptor blockade results in a slight stimulation in the prefrontal cortex (Merchant et al. Cerebral Cortex 1996, 6, 561-570), which could be beneficial against negative symptoms and cognitive deficits associated with schizophrenia. In addition, D₃ antagonists can reverse D₂ antagonist-induced EPS (Millan et al. Eur. J. Pharmacol. 1997, 321, R7-R9) and do not cause changes in prolactin (Reavill et al. J. Pharmacol. Exp. Ther. 2000, 294, 1154-1165). Consequently, D₃ antagonistic properties of an antipsychotic drug could reduce the negative symptoms and cognitive deficits and result in an improved side effect profile with respect to EPS and hormonal changes.

Dopamine D₃ agonists have also been considered relevant in the treatment of schizophrenia (Wustow et al. Current Pharmaceutical Design 1997, 3, 391-404).

Various effects are known with respect to compounds, which are ligands at the different serotonin receptor subtypes. As regards the 5-HT_{2A} receptor, which was previously referred to as the 5-HT₂ receptor, the following effects have been reported, e.g.:

Antidepressive effect and improvement of the sleep quality (Meert et al. Drug. Dev. Res. 1989, 18, 119), reduction of the negative symptoms of schizophrenia and of extrapyramidal side effects caused by treatment with classical neuroleptics in schizophrenic patients (Gelders British J. Psychiatry 1989, 155 (suppl. 5), 33). Furthermore, selective 5-HT_{2A} antagonists could be effective in the prophylaxis and treatment of migraine (Scrip Report; "Migraine - Current trends in research and treatment"; PJB Publications Ltd.; May 1991) and in the treatment of anxiety (Colpart et al. Psychopharmacology 1985, 86, 303-305 and Perregaard et al. Current Opinion in Therapeutic Patents 1993, 1, 101-128).

Some clinical studies implicate the 5-HT₂ receptor subtype in aggressive behaviour. Further, atypical serotonin-dopamine antagonist neuroleptics have 5-HT₂ receptor antagonistic effect in addition to their dopamine blocking properties and have been reported to possess anti-aggressive behaviour (Connor et al. Exp. Opin. Ther. Patents. 1998, 8(4), 350-351).

Recently, evidence has also accumulated which support the rational for selective 5-HT_{2A} antagonists as drugs capable of treating positive symptoms of psychosis (Leysen et al. Current Pharmaceutical Design 1997, 3, 367-390 and Carlsson Current Opinion in CPNS Investigational Drugs 2000, 2(1), 22-24).

Compounds, which are 5-HT reuptake inhibitors, are well-known antidepressant drugs.

5-HT_{2C} ligands have been found to augment the effect of 5-HT reuptake inhibitors in microdialysis experiments and animal models, and compounds having 5-HT reuptake inhibiting effect combined with affinity for the 5-HT_{2C} receptor may therefore be particularly useful for the treatment of depression and other disorders responsive to serotonin reuptake inhibitors (PCT application No. PCT/DK00/00671).

Accordingly, dopamine D₄ receptor ligands are potential drugs for the treatment of schizophrenia and other psychoses, and compounds with combined effects at the 5-HT transporter may have the further benefit of improved effect on depressive and negative symptoms in schizophrenic patients. Compounds with combined effect at the dopamine D₄ receptor and the 5-HT_{2A} receptor may have the benefit of improved effect on positive and negative symptoms of schizophrenia, and the benefit of effect on depressive and anxiety symptoms. Furthermore, dopamine D₃ antagonistic properties of an antipsychotic drug may reduce the negative symptoms and cognitive deficits of schizophrenia and result in an improved side effect profile.

### Summary of the Invention

The object of the present invention is to provide compounds that are partial agonists or antagonists at the dopamine D₄ receptor and such compounds with combined effects at the dopamine D₄ receptor, the D₃ receptor, the 5-HT_{2A} receptor, the 5-HT_{2C} receptor and/or the 5-HT transporter.

A further object of the present invention is to provide compounds with such activities which have improved solubility compared to prior art compounds.

Accordingly, the present invention relates to novel compounds of formula I 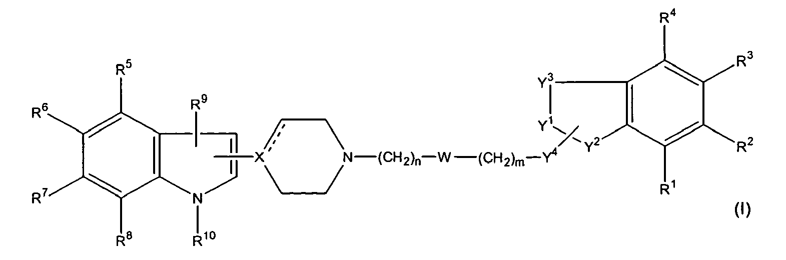 wherein
one of Y¹ and Y² is N, which is bound to Y⁴, and the other of Y¹ and Y² is CH₂ and Y⁴ is CO, CS, SO or SO₂;
Y³ is Z-CH₂, CH₂-Z or CH₂CH₂, and Z is O or S; provided that when Y¹ is N, Y³ may not be Z-CH₂;
W is a bond or an O, S, CO, CS, SO or SO₂ group;
n is 0-5, m is 0-5 and m + n is 1-10; provided that when W is O or S, then n ≥ 2 and m ≥ 1; when W is CO, CS, SO or SO₂, then n ≥ 1 and m ≥ 1;
X is C, CH or N; provided that when X is C, the dotted line indicates a bond, and when X is N or CH, the dotted line is not a bond;
R¹-R⁹ are independently selected from hydrogen, halogen, cyano, nitro, amino, hydroxy, C₁₋₆-alkyl-amino, di-C₁₋₆-alkyl-amino, C₁₋₆-alkyl, C₂₋₆-alkenyl, C₂₋₆-alkynyl, C₁₋₆-alkoxy, C₁₋₆-alkylthio, C₁₋₆-alkyl substituted with hydroxy or thiol, C₃₋₈-cycloalkyl, C₃₋₈-cycloalkyl-C₁₋₆-alkyl, acyl, thioacyl, aryl, trifluoromethyl, trifluoromethylsulfonyl, and C₁₋₆ alkylsulfonyl;
R¹⁰ is hydrogen, C₁₋₆-alkyl, C₂₋₆-alkenyl, C₂₋₆-alkynyl, C₁₋₆-alkyl substituted with hydroxy or thiol, , C₃₋₈-cycloalkyl, C₃₋₈-cycloalkyl-C₁₋₆-alkyl, aryl, aryl-C₁₋₆-alkyl, acyl, thioacyl, C₁₋₆-alkylsulfonyl, trifluoromethylsulfonyl or arylsulfonyl or a pharmaceutically acceptable acid addition salt thereof.

In a first particular embodiment of the invention, the indole is bound to X via position 3 of the indole.

In a second embodiment of the invention, one of Y¹ and Y² is N, which is bound to Y⁴, and the other of Y¹ and Y² is CH₂ and Y⁴ is CO..

In a further embodiment of the invention, Y¹ is a nitrogen bound to Y⁴, Y² is CH₂ and Y⁴ is CO.

In an further embodiment of the invention, Y² is a nitrogen atom bound to Y⁴, Y¹ is CH₂ and Y⁴ is CO.

Such compounds are preferably in the form of pharmaceutically acceptable di-salts thereof.

In a further embodiment of the invention, Y³ is CH₂CH₂ or CH₂Z.

In still further embodiments of the invention, X is C, X is N or X is CH

The substituents R¹-R⁹ are in particular selected from hydrogen, halogen, cyano, nitro, amino, C₁₋₆-alkylamino, di-C₁₋₆-alkylamino, C₁₋₆-alkyl, C₃₋₈-cycloalkyl and trifluoromethyl, and R¹⁰ is hydrogen, C₁₋₆-alkyl or acyl and /or W is a bond and n + m is 1 to 6, in particular 3 to 6.

The compounds of the invention are partial agonists or antagonist at the dopamine D₄ receptor. Many compounds have combined effect at the dopamine D₄ receptor and dopamine D₃ receptor affinity, 5-HT_{2A} receptor affinity, 5-HT_{2C} receptor affinity and /or 5-HT reuptake inhibiting effect.

Accordingly, the compounds of the invention are considered useful in the treatment of positive and negative symptoms of schizophrenia, other psychoses, anxiety disorders, such as generalised anxiety disorder, panic disorder, and obsessive compulsive disorder, depression, aggression, side effects induced by conventional antipsychotic agents, migraine, cognitive disorders, ADHD and in the improvement of sleep.

In another aspect, the present invention provides a pharmaceutical composition comprising at least one compound of Formula I as defined above or a pharmaceutically acceptable acid addition salt thereof in a therapeutically effective amount and in combination with one or more pharmaceutically acceptable carriers or diluents.

In a further aspect, the present invention provides the use of a compound of Formula I as defined above or an acid addition salt thereof for the manufacture of a pharmaceutical preparation for the treatment of the above mentioned disorders.

### Detailed Description of the Invention

The compounds of general Formula I may exist as optical isomers thereof and such optical isomers are also embraced by the invention.

The term C₁₋₆-alkyl refers to a branched or unbranched alkyl group having from one to six carbon atoms inclusive, such as methyl, ethyl, 1-propyl, 2-propyl, 1-butyl, 2-butyl, 2-methyl-2-propyl, 2-methyl-1-propyl, pentyl and hexyl.

Similarly, C₂₋₆-alkenyl and C₂₋₆-alkynyl, respectively, designate such groups having from two to six carbon atoms, including one double bond and triple bond respectively, such as ethenyl, propenyl, butenyl, ethynyl, propynyl and butynyl.

The terms C₁₋₆-alkoxy, C₁₋₆-alkylthio, C₁₋₆-alkylsulfonyl, C₁₋₆-alkylamino, C₁₋₆-alkylcarbonyl, and the like, designate such groups in which the alkyl group is C₁₋₆ alkyl as defined above.

The term C₃₋₈-cycloalkyl designates a monocyclic or bicyclic carbocycle having three to eight C-atoms, such as cyclopropyl, cyclopentyl, cyclohexyl, etc.

The term aryl refers to a carbocyclic aromatic group, such as phenyl, naphthyl, in particular phenyl, including methyl substituted phenyl, or naphthyl.

Halogen means fluoro, chloro, bromo or iodo.

As used herein the term acyl refers to a formyl, C₁₋₆-alkylcarbonyl, arylcarbonyl, aryl-C₁₋₆-alkylcarbonyl, C₃₋₈-cycloalkylcarbonyl or a C₃₋₈-cycloalkyl-C₁₋₆-alkyl-carbonyl group and the term thioacyl is the corresponding acyl group in which the carbonyl group is replaced with a thiocarbonyl group.

The acid addition salts of the compounds of the invention are pharmaceutically acceptable salts formed with non-toxic acids. Exemplary of such organic salts are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic and theophylline acetic acids, as well as the 8-halotheophyllines, for example 8-bromotheophylline. Exemplary of such inorganic salts are those with hydrochloric, hydrobromic, sulfuric, sulfamic, phosphoric and nitric acids.

The pharmaceutical compositions of this invention or those which are manufactured in accordance with this invention may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients, or other additives normally used in the art may be used.

Conveniently, the compounds of the invention are administered in unit dosage form containing said compounds in an amount of about 0.01 to 100 mg.

The total daily dose is usually in the range of about 0.05 - 500 mg, and most preferably about 0.1 to 50 mg of the active compound of the invention.

The compounds of the invention may be prepared as follows:
1) Alkylating a piperazine, piperidine or tetrahydropyridine of formula II with an alkylating derivative of formula III: 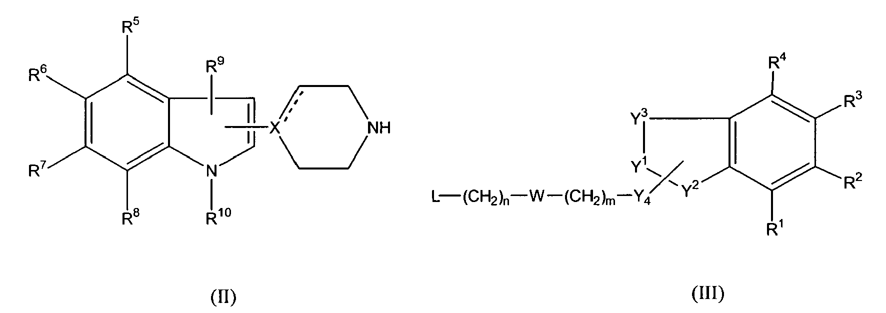 wherein R¹-R¹⁰, X, Y¹, Y², Y³, Y⁴, W, n, m and the dotted line are as previously defined, and L is a leaving group such as e.g. halogen, mesylate or tosylate;
2) Reductive alkylation of an amine of formula II with a reagent of formula IV: 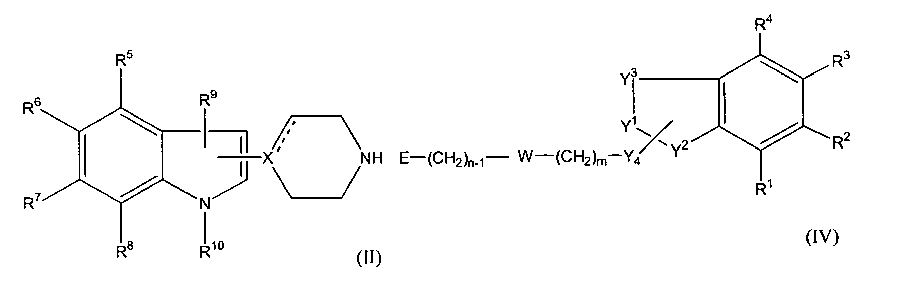 wherein R¹-R¹⁰, X, Y¹, Y², Y³, Y⁴, W, n, m and the dotted line are as previously defined and E is an aldehyde or an activated carboxylic acid group;
3) Alkylating a compound of formula V with an alkylating derivative of formula VI: 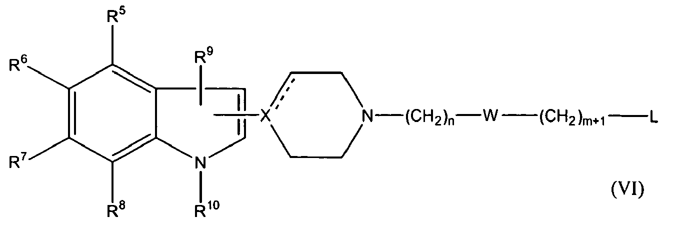 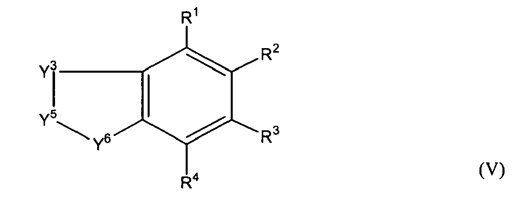 wherein R¹-R¹⁰, X, Y³, W, n, m and the dotted line are as previously defined, one of Y⁵ and Y⁶ is NH or N⁻ and the other of Y⁵ and Y⁶ is CO, CS, SO, SO₂ or CH₂ and L is a leaving group such as e.g. halogen, mesylate or tosylate; or
4) Reducing the double bond in the tetrahydropyridinyl ring in derivatives of the following formula VII: 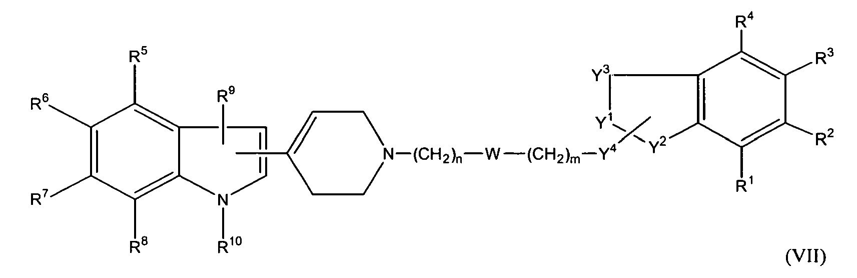 wherein R¹-R¹⁰, Y¹, Y², Y³, Y⁴, W, m and n are as previously defined;
5) Reducing the amide carbonyl in a compound of formula VIII: 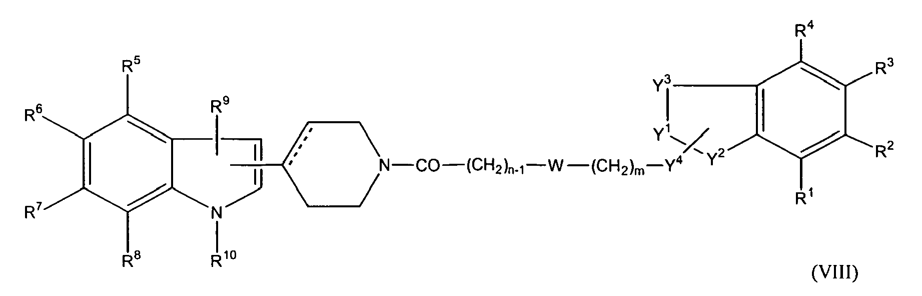 wherein R¹-R¹⁰, Y¹, Y², Y³, Y⁴, n, m, W and the dotted line are as previously defined;
6) Reducing the amide group compounds of formula IX: 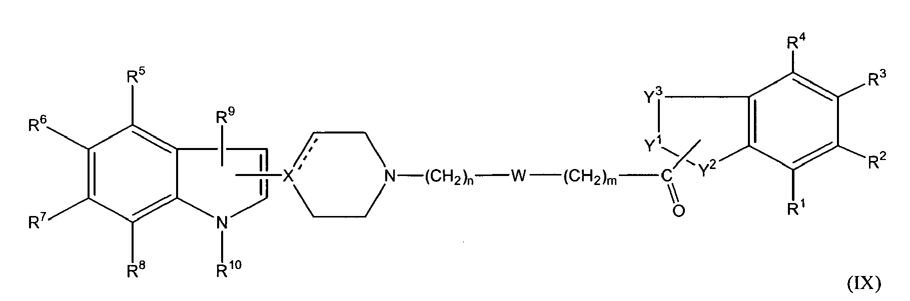 wherein R¹ - R¹⁰, X, Y¹, Y², Y³, n, m, W and the dotted line are as previously defined;
7) Reductive alkylation of a derivative of formula Va with an acylating derivative of formula X: 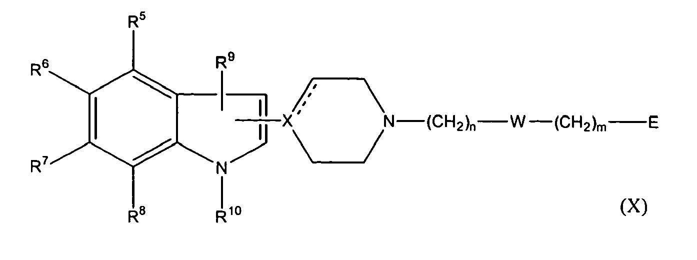 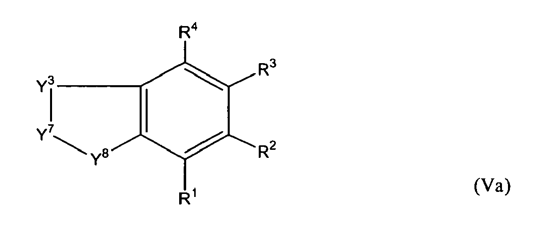 wherein R¹-R¹⁰, X, Y³, W, n, m and the dotted line are as previously defined, one of Y⁷ and Y⁸ is NH and the other of Y⁷ and Y⁸ is CH₂ and E is an aldehyde or an activated carboxylic acid;
8) Acylation of an amine of formula Va with a reagent of formula X: 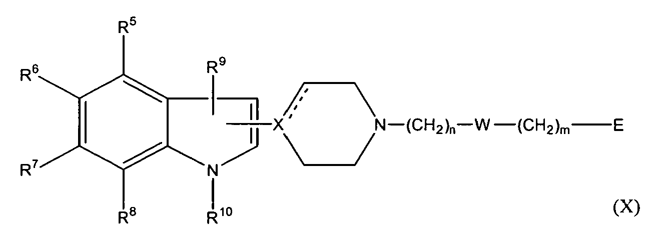 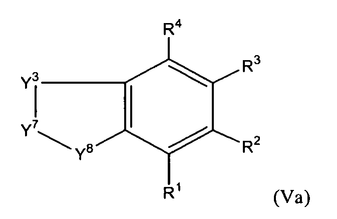 wherein R¹-R¹⁰, X, Y³, W, n, m and the dotted line are as previously defined, one of Y⁷ and Y⁸ is NH and the other of Y⁷ and Y⁸ is CH₂ and E is an aldehyde or an activated carboxylic acid;
9) Cleaving a polymer bound derivative of formula XI 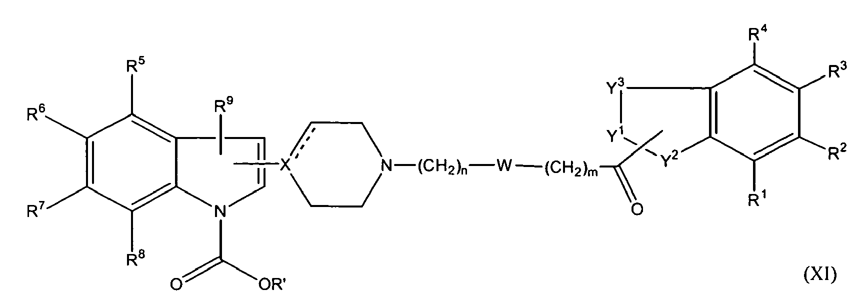 wherein R¹-R⁹, Y¹, Y², Y³, X, W, m and n are as previously defined and R'OH is hydroxyethyl or hydroxymethyl polystyrene, Wang resin or analogous polyethylene glycol polystyrene resins; whereupon the compound of Formula I is isolated as the free base or a pharmaceutically acceptable acid addition salt thereof.

The alkylation according to method 1) and 3) is conveniently performed in an inert organic solvent such as a suitably boiling alcohol or ketone, preferably in the presence of an organic or inorganic base (potassium carbonate, diisopropylethylamine or triethylamine) at reflux temperature. Alternatively, the alkylation can be performed at a fixed temperature, which is different from the boiling point, in one of the above-mentioned solvents or in dimethyl formamide (DMF), dimethylsulfoxide (DMSO) or *N*-methylpyrrolidin-2-one (NMP), preferably in the presence of a base.

The synthesis the amines of formula (II), 3-(piperidin-4-yl)-1*H*-indoles and 3-(3,6-dihydro-2*H*-pyridin-4-yl)-1*H*-indoles has been described in the literature (see e.g. EP-A1-465398). Alkylating reagents of formula (III) are known from the literature (see Oshiro et al. J. Med. Chem. 2000, 43, 177-189 and EP-B1-512525), or they can be prepared by methods obvious to a chemist skilled in the art (see e.g. Kowalski et al. J.Heterocyclic Chem. 2000, 37, 187-189, Mokrosz et al. Pharmazie 1997, 52, 423-428 and Misztal et al. Med.Chem.Res. 1992, 2, 82-87). Alkylating reagents of formula (VI) can be prepared by methods obvious to a chemist skilled in the art, and amines of formula (V) are commercially available or described in the literature.

The reductive alkylation according to methods 2) and 7) is performed by standard literature methods. The reaction can be performed in two steps, e.g. coupling of derivatives of formula II/Va and the reagent of formula IV/X by standard methods via the carboxylic acid chloride or by use of coupling reagents such as e.g. dicyclohexyl carbodiimide followed by reduction of the resulting amide with lithium aluminium hydride or alane. The reaction can also be performed by a standard one-pot procedure. Carboxylic acids or aldehydes of formula IV/X can be prepared by methods obvious to a chemist skilled in the art.

The alkylation according to method 3) is conveniently performed as described above or by reacting the nitrogen anion of V with VI. The nitrogen anion of V can be prepared in an inert organic solvent, e.g. dimethyl formamide (DMF), dimethylsulfoxide (DMSO) or *N*-methylpyrrolidin-2-one (NMP), by the use of a strong base, e.g. NaH, before the alkylation.

The reduction of the double bond according to method 4) is generally performed by catalytic hydrogenation at low pressure (< 3 atm.) in a Parr apparatus, or by using reducing agents such as diborane or hydroboric derivatives as produced *in situ* from NaBH₄ in trifluoroacetic acid in inert solvents such as tetrahydrofuran (THF), dioxane or diethyl ether. Starting materials of formula (VII) may be prepared by methods 1), 3), 7) and 8).

Reduction of amide groups according to methods 5) and 6) is most conveniently performed with lithium aluminium hydride or alane in an inert organic solvent such as e.g. tetrahydrofuran (THF) or diethylether from 0 °C to reflux temperature. Starting materials of formula (VIII) may be prepared by methods 2) and 3), whereas starting materials of formula (IX) may be prepared by methods 1), 7) and 8).

The coupling according to method 8) is conveniently performed by the use of coupling reagents such as e.g. dicyclohexyl carbodiimide.

The derivatives of structure (XI) is prepared by means of a solid phase synthesis sequence as outlined in Scheme 1 below. The first building block (XII), prepared by methods obvious to the chemist skilled in the art, is generally attached to the resin (polystyrene bound ethyl 4-nitrophenyl carbonate) using base e.g. *N,N* dimethylaminopyridine and *N,N*-diisopropylethylamine at elevated temperature (e.g. 50-100 °C) in an aprotic solvent (e.g.

DMF or DMSO) to yield (XIII). After deprotection of the amino group by trifluoroacetic acid (resin XIV), the second diversifying building block was introduced by alkylation. The alkylation was performed at elevated temperature (50-100 °C) in an aprotic solvent such as DMF, acetone or acetonitrile leading to resin (XV). After deprotection of the carboxylic acid ester by trifluoroacetic acid (resin XVI), the third diversifying building block of formula (Va) was introduced by standard amide forming reaction sequence, e.g. converting the carboxylic acid to the corresponding acid chloride using thionyl chloride at low temperature in dichloromethane, acetonitrile or DMF followed by treatment with an amine. The final product was cleaved from the resin using diluted sodium methoxide in a methanol/tetrahydrofuran mixture at ambient temperature.

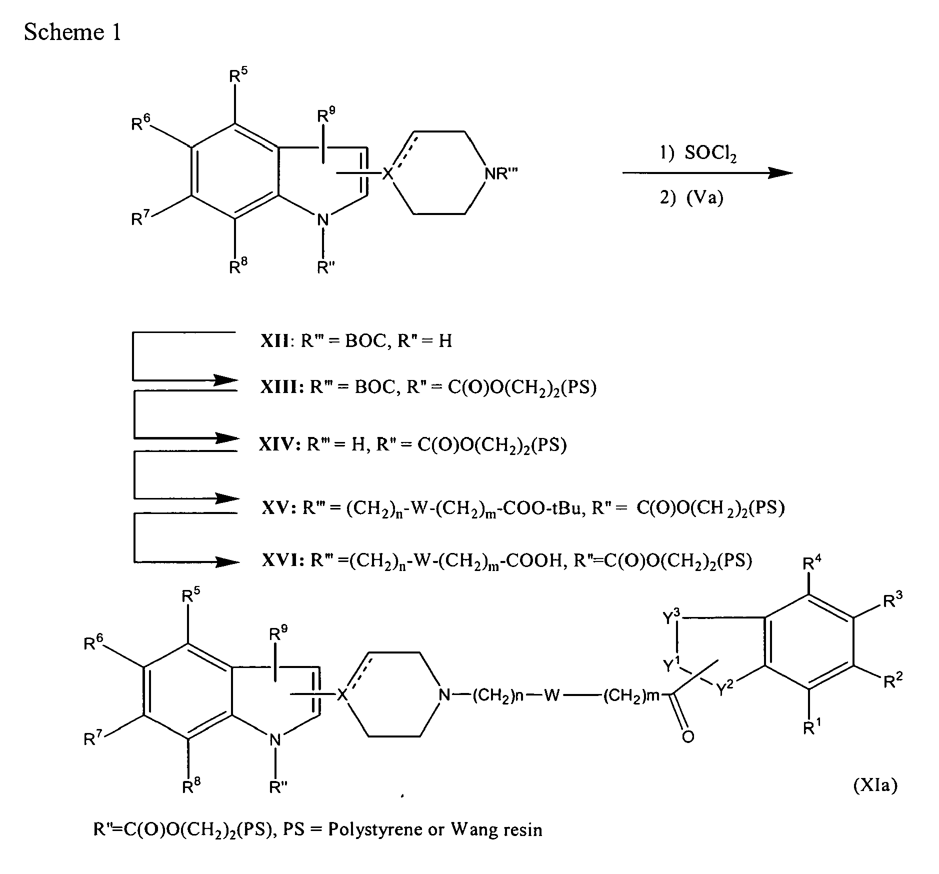

### Experimental Section

Melting points were determined on a Büchi B-540 apparatus and are uncorrected. Mass spectra were obtained on a Quattro MS-MS system from VG Biotech, Fisons Instruments. Analytical LC-MS data were obtained on a PE Sciex API 150EX instrument equipped with IonSpray source and Shimadzu LC-8A/SLC-10A LC system. The LC conditions (50 X 4.6 mm YMC ODS-A with 5 µm particle size) were linear gradient elution with water/acetonitrile/trifluoroacetic acid (90:10:0.05) to water/acetonitrile/trifluoroacetic acid (10:90:0.03) in 7 min at 2 mL/min. Purity was determined by integration of the UV trace (254 nm). The retention times Rₜ are expressed in minutes. Preparative LC-MS-separation was performed on the same instrument. The LC conditions (50 X 20 mm YMC ODS-A with 5 µm particle size) were linear gradient elution with water/acetonitrile/trifluoroacetic acid (80:20:0.05) to water/acetonitrile/trifluoroacetic acid (5:95:0.03) in 7 min at 22.7 mL/min. Fraction collection was performed by split-flow MS detection.
¹H NMR spectra were recorded at 250.13 MHz on a Bruker AC 250 or at 500.13 MHz on a Bruker DRX 500. Deuterated chloroform (99.8% D) or dimethylsulfoxide (99.9% D) were used as solvents. TMS was used as internal reference standard. Chemical shifts are expressed as ppm values. The following abbreviations are used for multiplicity of NMR signals: s=singlet, d=doublet, t=triplet, q=quartet, qv=quintet, h=heptet, dd=double doublet, dt=double triplet, dq=double quartet, tt=triplet of triplets, m= multiplet, b=broad. NMR signals corresponding to acidic protons are to some extent omitted. Content of water in crystalline compounds was determined by Karl Fischer titration. For column chromatography, silica gel of type Kieselgel 60, 40-60 mesh ASTM was used. For ionexchange chromatography, the following material was used: SCX-columns (1 g) from Varian Mega Bond Elut®, Chrompack cat. No. 220776. Prior to use, the SCX-columns were pre-conditioned with 10% solution of acetic acid in methanol (3 mL).

### Examples

### Preparation of intermediates

### A. Alkylating reagents

### 1-(2-Chloroethyl)-3,4-dihydroquinolin-2(1H)-one

A suspension of sodium hydride (3.0 g, 60% in mineral oil) and dimethyl formamide (100 mL) was kept at 15-18 °C followed by the addition of a solution of 3,4-dihydroquinolin-2(1*H*)-one (10.0 g) in dimethyl formamide (150 mL). The resulting mixture was stirred at room temperature for 60 min followed by the addition of a solution of 2-chloroethyl acetate (10.0 g) in dimethyl formamide (50 mL) at a temperature of 20 °C. The resulting mixture was heated at 80°C for 2 ½h, cooled and poured onto ice. The aqueous phase was extracted with ethyl acetate, and the combined organic phases were washed with brine, dried (MgSO₄) and concentrated *in vacuo.* The crude product was purified by flash chromatography on silicagel (eluent: ethyl acetate/heptane 1:1) to give crude 1-(2-acetoxyethyl)-3,4-dihydroquinolin-2(1*H*-one (10.2 g). A mixture of crude 1-(2-acetoxyethyl)-3,4-dihydroquinolin-2(1*H*)-one, sodium methanolate (2.5 mL, 30% in methanol) and methanol (250 mL) was stirred at room temperature for 16 h and subsequently concentrated *in vacuo.* The residue was purified by flash chromatography on silicagel (eluent: ethyl acetate/heptane 1: 1) to give the corresponding alcohol as a red crystalline compound (4.9 g). This alcohol was dissolved in tetrahydrofuran (100 mL) followed by the addition of trietylamine (8.2 mL). The resulting mixture was cooled to 5-6 °C followed by the addition of a solution of methane sulfonic acid chloride (2 mL) in tetrahydrofuran (25 mL). The mixture was filtered and evaporated to dryness *in vacuo.* The residue was dissolved in dimethyl formamide (50 mL) followed by addition of lithium chloride (4.9 g), and the resulting mixture was heated at 70 °C for 5 min. The mixture was poured onto brine, and the aqueous phase was extracted with ethyl acetate. The combined organic phases were dried (MgSO₄), filtered and concentrated *in vacuo.* The residue was purified by flash chromatography on silicagel (eluent: ethyl acetate/heptane 1:1) to give the product as a red oil (2.9 g).

### 1-(3-Bromopropan-1-yl)-3,4-dihydroquinolin-2(1H)-one

A suspension of sodium hydride (6.8 g, 60% in mineral oil) and dimethyl formamide (200 mL) was kept at 20-25 °C followed by the addition of a solution of 3,4-dihydroquinolin-2(1*H*)-one (25.0 g) in dimethyl formamide (180 mL). The resulting mixture was stirred at room temperature for 10 min followed by the addition of a solution of 1,3-dibromopropane (172 g) in dimethyl formamide (150 mL) at a temperature of 20-35 °C. The resulting mixture was stirred at 30 °C for 20 min and concentrated *in vacuo.* The residue was poured onto ice, and the aqueous phase was extracted with ethyl acetate. The combined organic phases were washed with brine, dried (MgSO₄) and concentrated *in vacuo.* The crude product was purified by flash chromatography on silicagel (eluent: ethyl acetate/heptane 1:1) to give the product as a yellow oil (27 g).

The following compounds were prepared in a similar manner
1-(4-Bromobutan-1-yl)-3,4-dihydroquinolin-2(1*H*)-one
   from 3,4-dihydroquinolin-2(1*H*)-one and 1,4-dibromobutane
1-(5-Bromopentan-1-yl)-3,4-dihydroquinolin-2(1*H*)-one
   from 3,4-dihydroquinolin-2(1*H*)-one and 1,5-dibromopentane
4-(4-Bromobutan-1-yl)-3,4-dihydro-2*H*-1,4-benzoxazin-3(4*H*)-one
   from 3,4-dihydro-2*H*-1,4-benzoxazin-3(4*H*)-one and 1,4-dibromobutane
2-(3-Hydroxypropan-1-yl)-3,4-dihydroisoquinolin-1(2*H*)-one
   from 3,4-dihydroisoquinolin-1(2*H*)-one and 3-bromopropanol
2-(4-Bromobutan-1-yl)-3,4-dihydroisoquinolin-1(2*H*)-one
   from 3,4-dihydroisoquinolin-1(2*H*)-one and 1,4-dibromobutane

### 1-(3-Bromopropan-1-yl)-3,4-dihydroisoquinolin-1(2H)-one

The compound 2-(3-hydroxypropan-1-yl)-3,4-dihydroisoquinolin-1(2*H*)-one was dissolved in tetrahydrofuran (100 mL) followed by the addition of triethylamine (5.2 mL). The resulting mixture was cooled to 6-11 °C followed by the addition of a solution of methane sulfonic acid chloride (1.4 mL) in tetrahydrofuran (25 mL). The mixture was stirred at 5 °C for 10 min, filtered and concentrated *in vacuo.* The residue was dissolved in acetone (250 mL) followed by addition of lithium bromide (6.5 g), and the resulting mixture was boiled under reflux for 2 h. The mixture was poured onto brine, and the aqueous phase was extracted with ethyl acetate. The combined organic phases were dried (MgSO₄), filtered and concentrated *in vacuo.* The residue was purified by flash chromatography on silicagel (eluent: ethyl acetate/heptane 1:2) to give the product as a yellow oil (2.7 g).

### 3-Chloro-1-(3,4-dihydro-1H-isoquinolin-2-yl)propan-1-one

A solution of 3-chloropropanoyl chloride (10.5 g) in tetrahydrofuran (400 mL) was cooled down to 6 °C followed by the addition of a solution of 3,4-dihydro-1*H*-isoquinoline (10.0 g). The resulting mixture was stirred at 10 °C for 30 min, filtered and concentrated *in vacuo.* The residue was subjected to a standard aqueous work up procedure followed by purification by flash chromatography on silicagel (eluent: ethyl acetate/heptane 1:1) to give the product as a colourless oil (10 g).

The following compounds were prepared in a similar manner
3-Bromo-1-(3,4-dihydro-1*H*-isoquinolin-2-yl)propan-1-one
   from 3,4-dihydro-1*H*-isoquinoline and 3-bromopropanoyl chloride
4-Chloro-1-(3,4-dihydro-1*H*-isoquinolin-2-yl)butan-1-one
   from 3,4-dihydro-1*H*-isoquinoline and 4-chlorobutanoyl chloride
4-Chloro-1-(3,4-dihydro-2*H*-quinolin-1-yl)butan-1-one
   from 3,4-dihydro-2*H*-quinoline and 4-chlorobutanoyl chloride

### Preparation of solid supported intermediates

### Preparation of 4-nitrophenyloxycarbonyloxyethyl polystyren

A 2 L round bottom flask was charged with hydroxyethyl polystyren (62.9 g, 83 mmol, commercially available from Rapp Polymere, cat. no. HA 1 400 00), *N*-methyl-morpholine (20 mL, 183 mmol) and dry dichloromethane (900 mL). The suspension was cooled on an ice bath and 4-nitrophenyl chloroformiate dissolved in dry dichloromethane (400 mL) was added during 5 minutes. The mixture was stirred at room temperature for 16 h. The resin was filtered off and washed with dry dichloromethane (5 × 200 mL). The resin was dried in *vacuo* (20 °C, 72 h) to yield the title resin (79.6 g).

### Preparation of polymer bound 7-chloro-3-(piperidin-4-yl)-1H-indole

A 100 mL round bottom flask was charged with 4-nitrophenyloxycarbonyloxyethyl polystyren (4.0 g, 4.3 mmol), 7-chloro-3-(1-*tert*-butoxycarbonylpiperidin-4-yl)-1*H*-indole (2.7 g, 8.1 mmol), diisopropylethylamine (3.5 mL, 20.2 mmol), 4-dimethylaminopyridine (0.5 g, 4 mmol) and dry dimethyl formamide (50 mL). The mixture was stirred at 90 °C for 72 h. After cooling to room temperature, the resin was filtered off and washed with dry dimethyl formamide (3 × 25 mL), dry acetonitrile (3 × 25 mL) and dry dichloromethane (3 × 25 mL). The resin was transferred to a 250 mL glass cylinder with a fritte and a three way junction in the bottom. The resin was then treated for 20 minutes with 60 mL of a 1:1 mixture of dichloromethane and trifluoroacetic acid containing anisole (2%, w/w) and methionine (0.2 %, w/w), using a flow of nitrogen to agitate the resin (Caution: Generation of carbon dioxide). The resin was filtered off and washed with dry dichloromethane (25 mL), a 1:1 mixture of dichloromethane:triethylamine (3 × 25 mL) and dry dichloromethane (3 × 25 mL). The resin was dried *in vacuo* (20 °C, 20 h) to yield the title resin (3.8 g).

The following polymer bound compounds were prepared in a similar manner
4-Chloro-3-(piperidin-4-yl)-1*H*-indole
4-Fluoro-3-(piperidin-4-yl)-1*H*-indole
5-Chloro-3-(piperidin-4-yl)-1*H*-indole
5-Fluoro-3-(piperidin-4-yl)-1*H*-indole
6-Chloro-3-(piperidin-4-yl)-1*H*-indole

### Preparation of polymer bound 3-[4-(7-chloro-1H indol-3-yl)piperidin-1-yl]propionic acid

A 25 mL round bottom flask was charged with polymer bound 7-chloro-3-(piperidin-4-yl)-1*H*-indole (1.0 g, 0.98 mmol), triethylamine (80.2 mL), *tert*-butyl 3-bromopropionate and dry acetonitrile (5 mL). The mixture was stirred at 80 °C for 3 h. After cooling to room temperature, the resin was filtered off and washed with dry acetonitrile (3 × 10 mL) and dry dichloromethane (3 × 10 mL). The resin was treated for 20 minutes with 8 mL of a 1:1 mixture of dichloromethane and trifluoroacetic acid containing anisole (2%, w/w) and methionine (0.2 %, w/w) (Caution: Generation of carbon dioxide). The resin was filtered off and washed with dry dichloromethane (10 mL), a 1:1 mixture of dichloromethane:triethylamine (3 × 10 mL) and dry dichloromethane (3 × 10 mL). The resin was dried *in vacuo* (20°C, 20 h) to yield the title resin (1.0 g).

The following polymer bound compounds were prepared in a similar manner
3-[4-(4-Chloro-1*H*-indol-3-yl)piperidin-1-yl]propionic acid
3-[4-(4-Fluoro-1*H*-indol-3-yl)piperidin-1-yl]propionic acid
3-[4-(5-Fluoro-1*H*-indol-3-yl)piperidin-1-yl]propionic acid
3-[4-(6-Chloro-1*H*-indol-3-yl)piperidin-1-yl]propionic acid

4-[4-(4-Chloro-1*H*-indol-3-yl)piperidin-1-yl]butyric acid
4-[4-(4-Fluoro-1*H*-indol-3-yl)piperidin-1-yl]butyric acid
4-[4-(5-Chloro-1*H*-indol-3-yl)piperidin-1-yl]butyric acid
4-[4-(5-Fluoro-1*H*-indol-3-yl)piperidin-1-yl]butyric acid
4-[4-(7-Chloro-1*H*-indol-3-yl)piperidin-1-yl]butyric acid

5-[4-(4-Chloro-1*H*-indol-3-yl)piperidin-1-yl]pentanoic acid
5-[4-(5-Fluoro-1*H*-indol-3-yl)piperidin-1-yl]pentanoic acid
5-[4-(7-Chloro-1*H*-indol-3-yl)piperidin-1-yl]pentanoic acid

6-[4-(4-Fluoro-1*H*-indol-3-yl)piperidin-1-yl]hexanoic acid
6-[4-(4-Chloro-1*H*-indol-3-yl)piperidin-1-yl]hexanoic acid
6-[4-(5-Fluoro-1H-indol-3-yl)piperidin-1-yl]hexanoic acid
6-[4-(6-Chloro-1H-indol-3-yl)piperidin-1-yl]hexanoic acid
6-[4-(7-Chloro-1H-indol-3-yl)piperidin-1-yl]hexanoic acid

### Preparation of the compounds of the disclosure

### Example 1

### 1a, 5-Fluoro-3-{1-[2-(2-oxo-3,4-dihydro-2H-quinolin-1-yl)ethyl]piperidin-4-yl}-1H-indole, hydrochloride

A mixture of 5-fluoro-3-(piperidin-4-yl)-1*H*-indole (0.3 g), 1-(2-chloroethyl)-3,4-dihydroquinolin-2(1*H*)-one (0.41 g) and triethylamine (0.75 g) in dimethyl formamide (5 mL) and butanone (10 mL) was boiled under reflux for 6 h. The mixture was concentrated *in vacuo,* and the residue was purified by flash chromatography on silicagel (eluent: ethyl acetate/ethanol/triethylamine 90:10:5) to give the crude product, which was isolated as the hydrochloride salt from acetone as a white crystalline compound (0.04 g). ¹H NMR (DMSO-d₆): 2.00-2.25 (m, 4H); 2.60 (t, 2H); 2.90 (t, 2H); 2.95-3.10 (m, 1H); 3.10-3.30 (m, 4H); 3.70 (d, 2H); 4.35 (t, 2H); 6.90 (t, 1H); 7.05 (t, 1H); 7.15-7.40 (m, 5H); 7.50 (d, 1H); 10.95 (broad s, 1H); 11.05 (s, 1H). MS m/z: 392 (MH+), 174.

The following compounds were prepared in a similar manner

### 1b, 5-Fluoro-3-{1-[3-(1-oxo-3,4-dihydro-1H-quinolin-2-yl)propan-1-yl]piperidin-4-yl}-1H-indole, oxalate

from 5-fluoro-3-(piperidin-4-yl)-1*H*-indole and 1-(3-bromopropan-1-yl)-3,4-dihydroisoquinolin-1(2*H*)-one. ¹H NMR (DMSO-d₆): 1.90-2.15 (m, 6H); 2.95-3.15 (m, 7H); 3.55-3.60 (m, 6H); 6.90 (t, 1H); 7.20 (s, 1H); 7.30 (d, 1H); 7.30-7.40 (m, 4H); 7.45-7.50 (m, 1H); 7.90 (d, 1H); 11.05 (s, 1H). MS m/z: 406 (MH+), 188.

### 1c, 5-Fluoro-3-{1-[4-(1-oxo-3,4-dihydro-1H-quinolin-2-yl)butan-1-yl]piperidin-4-yl}-1H-indole, hydrochloride

from 5-fluoro-3-(piperidin-4-yl)-1*H*-indole and 2-(4-bromobutan-1-yl)-3,4-dihydroisoquinolin-1(2*H*)-one. ¹H NMR (DMSO-d₆): 1.55-1.70 (m, 2H); 1.70-1.85 (m, 2H); 2.05 (d, 2H); 2.10-2.25 (m, 2H); 2.90-3.15 (7H); 3.40- 3.65 (m, 6H); 6.90 (t, 1H); 7.20 (s, 1H); 7.30 (d, 1H); 7.30-7.40 (m, 2H); 7.40-7.55 (m, 2H); 7.90 (d, 1H); 10.75 (broad s, 1H); 11.05 (s, 1H). MS m/z: 420 (MH+).

### Example 2

### 2a, 5-Fluoro-3-{1-[3-(2-oxo-3,4-dihydro-2H-quinolin-1-yl)propan-1-yl]piperidin-4-yl}-1H- indole, hydrochloride

A mixture of 5-fluoro-3-(piperidin-4-yl)-1*H*-indole (5.0 g), 1-(3-bromopropan-1-yl)-3,4-dihydroquinolin-2(1*H*)-one (7.7 g) and potassium carbonate (7.0 g) in dimethyl formamide (40 mL) was heated at 100 °C for 2½ h. The mixture was cooled, filtered and concentrated *in vacuo.* The residue was purified by flash chromatography on silicagel (eluent: ethyl acetate followed by ethyl acetate/ethanol 90:10) to give the product as an orange oil (9.1 g). The title compound (1.8 g of free base) was isolated as the hydrochloride salt from tetrahydrofuran as a white crystalline compound (1.5 g). Mp 210-212 °C. ¹H NMR (DMSO-d₆): 2.00-2.20 (m, 6H); 2.60 (t, 2H); 2.90 (t, 2H); 2.95-3.10 (m, 3H); 3.10-3.20 (m, 2H); 3.55 (d, 2H); 3.95 (t, 2H); 6.90 (t, 1H); 7.05 (t, 1H); 7.15-7.30 (m, 4H); 7.30-7.40 (m, 1H); 7.50 (d, 1H); 10.55 (broad s, 1H); 11.05 (s, 1H). MS m/z: 406 (MH+).

The following compounds were prepared in a similar manner

### 2b, 5-Fluoro-3-{1-[5-(2-oxo-3,4-dihydro-2H-quinolin-1-yl)pentan-1-yl]piperidin-4-yl}-1H-indole, hydrochlori de

from 5-fluoro-3-(piperidin-4-yl)-1*H*-indole and 1-(5-bromopentan-1-yl)-3,4-dihydroquinolin-2(1*H*)-one. Mp 199-200 °C. ¹H NMR (DMSO-d₆): 1.30-1.40 (m, 2H); 1.55-1.60 (m, 2H); 1.70-1.80 (m, 2H); 2.05-2.15 (m, 4H); 2.55 (t, 2H); 2.85 (t, 2H); 2.95-3.10 (m, 5H); 3.55 (d, 2H); 3.90 (t, 2H); 6.90 (t, 1H); 7.00 (t, 1H); 7.15 (d, 1H); 7.20-7.30 (m, 3H); 7.30-7.35 (m, 1H); 7.50 (d, 1H); 12.20 (broad s, 1H); 11.05 (s, 1H). MS m/z: 434 (MH+).

### 2c, 5-Chloro-3-{1-[3-(2-oxo-3,4-dihydro-2H-quinolin-1-yl)propan-1-yl]piperidin-4-yl}-1H- indole, hydrochloride

from 5-chloro-3-(piperidin-4-yl)-1*H*-indole and 1-(3-bromopropan-1-yl)-3,4-dihydroquinolin-2(1*H*)-one. Mp 142-146 °C. ¹H NMR (DMSO-d₆): 1.95-2.15 (m, 6H); 2.60 (t, 2H); 2.90 (t, 2H); 2.95-3.15 (3H); 3.15-3.20 (m, 2H); 3.55 (d, 2H); 3.95 (t, 2H); 7.00-7.10 (m, 2H); 7.20-7.30 (m, 4H); 7.35 (d, 1H); 7.75 (s, 1H), 11.30 (broad s, 1H); 11.15 (s, 1H). MS m/z: 422 (MH+), 188.

### 2d, 5-Chloro-3-{1-[4-(2-oxo-3,4-dihydro-2H-quinolin-1-yl)butan-1-yl]piperidin-4-yl}-1H-indole, hydrochloride

from 5-chloro-3-(piperidin-4-yl)-1*H*-indole and 1-(4-bromobutan-1-yl)-3,4-dihydroquinolin-2(1*H*)-one. Mp 229-231 °C. ¹H NMR (DMSO-d₆): 1.55-1.65 (m, 2H); 1.70-1.80 (m, 2H); 2.00-2.15 (m, 4H); 2.55 (t, 2H); 2.85 (t, 2H); 2.95-3.15 (m, 5H); 2.55 (d, 2H); 3.95 (t, 2H); 7.00 (t, 1H); 7.05 (d, 1H); 7.15 (d, 1H); 7.20-7.30 (m, 3H); 7.40 (d, 1H); 7.75 (s, 1H); 10.05 (broad s, 1H); 11.10 (s, 1H). MS m/z: 436 (MH+).

### 2e, 5-Chloro-3-{1-[5-(2-oxo-3,4-dihydro-2H-quinolin-1-yl)pentan-1-yl]piperidin-4-yl}-1H- indole, hydrochloride

from 5-chloro-3-(piperidin-4-yl)-1*H*-indole and 1-(5-bromopentan-1-yl)-3,4-dihydroquinolin-2(1*H*)-one. Mp 206-209 °C. ¹H NMR (DMSO-d₆): 1.30-1.40 (m, 2H); 1.55-1.65 (m, 2H); 1.70-1.80 (m, 2H); 2.00-2.15 (m, 4H); 2.55 (t, 2H); 2.85 (t, 2H); 2.95-3.10 (m, 4H); 3.10-3.25 (m, 1H); 3.55 (d, 2H); 3.90 (t, 2H); 7.00 (t, 1H); 7.05 (d, 1H); 7.15 (d, 1H); 7.20-7.30 (m, 3H); 7.40 (d, 1H); 7.75 (s, 1H); 11.20 (broad s, 1H); 11.15 (s, 1H). MS m/z: 450 (MH+), 299.

### 2f, 7-Chloro-3-{1-[4-(2-oxo-3,4-dihydro-2H-quinolin-1-yl)butan-1-yl]piperidin-4-yl}-1H-indole, hydrochloride

from 7-chloro-3-(piperidin-4-yl)-1*H*-indole and 1-(4-bromobutan-1-yl)-3,4-dihydroquinolin-2(1*H*)-one. Mp 253-254 °C. ¹H NMR (DMSO-d₆): 1.55-1.65 (m, 2H); 1.75-1.85 (m, 2H); 2.05-2.25 (m, 4H); 2.55 (t, 2H); 2.90 (t, 2H); 2.95-3.15 (m, 5H); 3.55 (d, 2H); 3.95 (t, 2H); 6.95-7.05 (m, 2H); 7.15-7.30 (m, 5H); 7.70 (d, 1H); 10.60 (broad s, 1H); 11.30 (s, 1H). MS m/z: 436 (MH+), 289.

### 2g, 5-Fluoro-3-{1-[4-(3-oxo-3,4-dihydro-2H-1,4-benzoxazin-4-yl)butan-1 yl]piperidin-4-yl}-1H-indole, hydrochloride

from 5-fluoro-3-(piperidin-4-yl)-1*H*-indole and 4-(4-bromobutan-1-yl)-3,4-dihydro-2*H*-1,4-benzoxazin-3(4*H*)-one. Mp 83-92 °C. ¹H NMR (DMSO-d₆): 1.60-1.70 (m, 2H); 1.75-1.85 (m, 2H); 2.00-2.20 (m, 4H); 2.95-3.15 (m, 5H); 3.55 (d, 2H); 3.95 (t, 2H); 4.65 (s, 2H); 6.90 (t, 1H); 7.00-7.05 (m, 2H); 7.05-7.15 (m, 1H); 7.20 (s, 1H); 7.25 (d, 1H); 7.30-7.40 (m, 1H); 7.50 (d, 1H); 10.45 (broad s, 1H); 11.05 (s, 1H). MS m/z: 422 (MH+), 273.

### 2h, 5-Chloro-3-{1-[4-(3-oxo-3,4-dihydro-2H-1,4-benzoxazin-4-yl)butan-1-yl]piperidin-4-yl}-1H-indole, hydrochloride

from 5-chloro-3-(piperidin-4-yl)-1*H*-indole and 4-(4-bromobutan-1-yl)-3,4-dihydro-2*H*-1,4-benzoxazin-3(4*H*)-one. Mp 222-224 °C. ¹H NMR (DMSO-d₆): 1.60-1.70 (m, 2H); 1.75-1.85 (m, 2H); 2.05-2.15 (m, 4H); 3.00-3.15 (m, 5H); 3.55 (d, 2H); 3.95 (t, 2H); 4.65 (s, 2H); 7.00-7.10 (m, 4H); 7.20 (s, 1H); 7.25 (d, 1H); 7.40 (d, 1H); 7.75 (s, 1H); 10.30 (broad s, 1H); 11.15 (s, 1H). MS m/z: 438 (MH+), 291, 204.

### Example 3

### 3a, 5-Fluoro-3-{1-[3-(2-oxo-3,4-dihydro-2H-quinolin-1-yl)propan-1-yl]-3,6-dihydro-2H-pyridin-4-yl}-1H-indole, oxalate

A mixture of 5-fluoro-3-(3,6-dihydro-2*H*-pyridin-4-yl)-1*H*-indole (3.0 g) and potassium carbonate (6.2 g) in butanone (250 mL) was heated until reflux temperature followed by the addition of 1-(3-bromopropan-1-yl)-3,4-dihydroquinolin-2(1*H*)-one (5.0 g) in butanone (50 mL). The resulting mixture was boiled under reflux for 10 h, filtered and concentrated *in vacuo* (7.7 g). The residue was purified by flash chromatography on silicagel (eluent: ethyl acetate/triethylamine 100:5) to give the crude product, which was crystallized from tetrahydrofuran/ethyl acetate. The title compound was isolated as the oxalate salt from acetone/tetrahydrofuran as a yellowish crystalline compound (1.7 g). Mp 203-206 °C. ¹H NMR (DMSO-d₆): 1.95-2.05 (m, 2H); 2.55 (t, 2H); 2.75 (s, 2H); 2.85 (t, 2H); 3.15 (t, 2H); 3.35 (s, 2H); 3.80 (s, 2H); 3.95 (t, 2H); 6.05 (s, 1H); 6.95-7.05 (m, 2H); 7.15-7.30 (m, 3 H); 7.35-7.45 (m, 1H); 7.50-7.60 (m, 2H); 11.50 (s, 1H). MS m/z: 404 (MH+), 218.

The following compounds were prepared in a similar manner

### 3b, 5-Fluoro-3-{1-[4-(2-oxo-3,4-dihydro-2H-quinolin-1-yl)butan-1-yl]-3,6-dihydro-2H- pyridin-4-yl}-1H-indole, hydrochloride

from 5-fluoro-3-(3,6-dihydro-2*H*-pyridin-4-yl)-1*H*-indole and 1-(4-bromobutan-1-yl)-3,4-dihydroquinolin-2(1*H*)-one. Mp 124-125 °C. ¹H NMR (DMSO-d₆): 1.55-1.65 (m, 2H); 1.80 (q, 2H); 2.55 (t, 2H); 2.75 (d, 1H); 2.85-2.95 (m, 3H); 3.15-3.30 (m, 3H); 3.55-3.65 (m, 1H); 3.75 (d, 1H); 3.90-4.00 (m, 3H); 6.10 (s, 1H); 6.95-7.05 (m, 2H); 7.15 (d, 1H); 7.20-7.30 (m, 2H); 7.40-7.45 (m, 1H); 7.55-7.65 (m, 2H); 10.70 (broad s, 1H); 11.50 (s, 1H). MS m/z: 418 (MH+), 231.

### 3c, 5-Fluoro-3-{1-[5-(2-oxo-3,4-dihydro-2H-quinolin-1-yl)pentan-1-yl]-3,6-dihydro-2H-pyridin-4-yl}-1H-indole, oxalate

from 5-fluoro-3-(3,6-dihydro-2*H*-pyridin-4-yl)-1*H*-indole and 1-(5-bromopentan-1-yl)-3,4-dihydroquinolin-2(1*H*)-one. Mp 205-207 °C. ¹H NMR (DMSO-d₆): 1.35 (t, 2H); 1.55 (t, 2H); 1.75 (t, 2H); 2.55 (t, 2H); 2.75 (s, 2H); 2.85 (t, 2H); 3.10 (t, 2H); 3.35 (s, 2H); 3.80 (s, 2H); 3.90 (t, 2H); 6.10 (s, 1H); 6.95-7.05 (m, 2H); 7.15 (d, 1H); 7.20-7.30 (m, 2H); 7.40-7.45 (m, 1H); 7.55-7.60 (m, 2H); 11.50 (s, 1H). MS m/z: 432 (MH+), 245.

### Example 4

### 4, 5-Fluoro-3-{1-[4-(2-oxo-3,4-dihydro-2H-quinolin-1-yl)butan-1-yl]piperidin-4-yl}-1H- indole, hydrochloride

A mixture of 5-fluoro-3-{1-[4-(2-oxo-3,4-dihydro-2*H* quinolin-1-yl)butan-1-yl]-3,6-dihydro-2*H*-pyridin-4-yl}-1*H*-indole (3.5 g), ethanol (100 mL), acetic acid (100 mL) and platinum oxide (0.4 g) was shaken under 3 atm for 16 h. The mixture was filtered, evaporated *in vacuo* to about a 100 mL, which subsequently was poured onto ice and added aqueous ammonia to basic pH. The aqueous phase was extracted with ethyl acetate, and the combined organic phases were washed with brine, dried (MgSO₄), and concentrated *in vacuo.* The residue was purified by flash chromatography on silicagel (eluent: ethyl acetate/triethylamine 100:4) to give the crude product (2.0 g). The title compound was isolated as the hydrochloride salt from ethyl acetate as a white crystalline compound (2.0 g). Mp 212-213 °C. ¹H NMR (DMSO-d₆): 1.55-1.65 (m, 2H); 1.75-1.85 (m, 2H); 2.00-2.20 (m, 4H); 2.55 (t, 2H); 2.85 (t, 2H); 2.95-3.15 (m, 5H); 3.55 (d, 2H); 3.95 (t, 2H); 6.90 (t, 1H); 7.00 (t, 1H); 7.15-7.30 (m, 4H); 7.30-7.40 (m, 1H); 7.50 (d, 1H); 10.55 (broad s, 1H); 11.05 (s, 1H). MS m/z: 420 (MH+), 273, 202.

### Example 5

### 5a, 5-Fluoro-1-methyl-3-{1-[3-(2-oxo-3,4-dihydro-2H-quinolin-1-yl)propan-1-yl]piperidin-4 yl}-1H-indole, oxalate

A suspension of sodium hydride (0.5 g, 60% in mineral oil) and dimethyl formamide (60 mL) was kept at 22-24 °C followed by the addition of a solution of 5-fluoro-3-{1-[3-(2-oxo-3,4-dihydro-2*H*-quinolin-1-yl)propan-1-yl]piperidin-4-yl}-1*H*-indole (4.9 g) in dimethyl formamide (50 mL). The resulting mixture was stirred at room temperature for 25 min followed by the addition of a solution of methyl iodide (2.0 g) in dimethyl formamide (15 mL) at a temperature of 22-27 °C. The resulting mixture was stirred at 22 °C for 1 h and poured onto ice. The aqueous phase was extracted with ethyl acetate, and the combined organic phases were washed with brine, dried (MgSO₄) and concentrated *in vacuo.* The crude product was purified by flash chromatography on silicagel (eluent: ethyl acetate/heptane/triethylamine 50:50:5) to give the product as an orange oil (2.4 g). The title compound was isolated as the oxalate salt from acetone as a white crystalline compound (0.6 g). Mp 188-189 °C. ¹H NMR (DMSO-d₆): 1.85-2.05 (m, 4H); 2.10 (d, 2H); 2.55 (t, 2H); 2.90 (t, 2H); 2.95-3.05 (m, 3H); 3.10 (t, 2H); 3.50 (d, 2H); 3.75 (s, 3H); 3.95 (t, 2H); 6.95-7.05 (m, 2H); 7.15-7.30 (m, 4H); 7.35-7.45 (m, 2H). MS m/z: 420 (MH+), 188.

The following compounds were prepared in a similar manner

### 5b, 5-Fluoro-1-methyl-3-{1-[4-(2-oxo-3,4-dihydro-2H-quinolin-1-yl)butan-1-yl]piperidin-4-yl}-1H-indole, hydrochloride

from 5-fluoro-3-{1-[4-(2-oxo-3,4-dihydro-2*H*-quinolin-1-yl)butan-1-yl]piperidin-4-yl}-1*H*-indole and methyl iodide. Mp 177-179 °C. ¹H NMR (DMSO-d₆): 1.55-1.65 (m, 2H); 1.75-1.85 (m, 2H); 2.00-2.15 (m, 4H); 2.55 (t, 2H); 2.90 (t, 2H); 2.95-3.15 (m, 5H); 3.55 (d, 2H); 3.75 (s, 3H); 3.95 (t, 2H); 6.95-7.05 (m, 2H); 7.15 (d, 1H); 7.20-7.30 (m, 3H); 7.35-7.45 (m, 1H); 7.55 (d, 1H); 11.40 (broad s, 1H). MS m/z: 434 (MH+).

### 5c, 1-(Butan-1-yl)-5-fluoro-3-{1-[4-(2-oxo-3,4-dihydro-2H-quinolin-1-yl)butan-1-yl]-3,6-dihydro-2H-pyridin-4-yl}-1H-indole, oxalate

from 5-fluoro-3-{1-[4-(2-oxo-3,4-dihydro-2*H*-quinolin-1-yl)butan-1-yl]-3,6-dihydro-2*H*-pyridin-4-yl}-1*H*-indole and butyl bromide. Mp 152-154°C. ¹H NMR (DMSO-d₆): 0.90 (t, 3H); 1.20-1.30 (m, 2H); 1.55-1.65 (m, 2H); 1.65-1.80 (m, 4H); 2.55 (t, 2H); 2.75 (s, 2H); 2.85 (t, 2H); 3.10 (t, 2H); 3.35 (s, 2H); 3.80 (s, 2H); 3.95 (t, 2H); 4.15 (t, 2H); 6.10 (s, 1H); 6.95-7.05 (m, 2H); 7.15 (d, 1H); 7.20-7.30 (m, 2H); 7.50-7.55 (m, 1); 7.55-7.70 (m, 2H). MS m/z: 474 (MH+), 231.

### Example 6

### 6a, 5-Fluoro-3-{1-3-(3,4-dihydro-1H-isoquinolin-2-yl)-3-oxopropan-1-yl]piperidin-4 yl}-1H-indole, oxalate

A mixture of 5-fluoro-3-(piperidin-4-yl)-1H-indole (3.0 g), butanone (200 mL), tetrahydrofuran (100 mL), methanol (50 mL) and triethylamine (2.4 mL) was heated until reflux temperature followed by the addition of a solution of 3-chloro-1-(3,4-dihydro-1*H*-isoquinolin-2-yl)propan-1-one (3.5 g) in butanone (60 mL). The mixture was boiled under reflux for 30 h followed by the addition of an additional amount of 3-chloro-1-(3,4-dihydro-1*H*-isoquinolin-2-yl)propan-1-one (2.0 g) and triethylamine (1.6 mL) in tetrahydrofuran (50 mL). The resulting mixture was boiled under reflux for an additional 12 h. The mixture was cooled, filtered and concentrated *in vacuo.* The residue was purified by flash chromatography on silicagel (eluent: ethyl acetate/ethanol/triethylamine 100:4:4) to give the crude product. The title compound was isolated as the oxalate salt from acetone as a white crystalline compound (0.75 g). Mp 206-209 °C. ¹H NMR (DMSO-d₆): 1.95 (q, 2H); 2.05-2.15 (m, 2H); 2.80 (t, 0.8H); 2.90 (t, 1.2H); 2.90-3.10 (m, 5H); 3.30 (t, 2H); 3.55 (d, 2H); 3.70 (t, 2H); 4.65 (s, 1.20H); 4.70 (s, 0.8H); 6.85-6.95 (m, 1H); 7.15-7.25 (m, 5H); 7.30-7.40 (m, 1H); 7.40 (d, 1H); 11.05 (s, 1H). MS m/z: 406 (MH+), 231.

The following compound was prepared in a similar manner

### 6b, 7-Chloro-3-{1-[3-(3,4-dihydro-1H-isoquinolin-2-yl)-3-oxopropan-1-yl]piperidin-4-yl}- 1H-indole, hydrochloride

from 7-chloro-3-(piperidin-4-yl)-1*H*-indole and 3-bromo-1-(3,4-dihydro-1*H*-isoquinolin-2-yl)propan-1-one. ¹H NMR (DMSO-d₆): 2.05-2.25 (m, 4H); 2.80 (t, 0.8H); 2.95 (t, 1.2H); 3.00-3.20 (m, 5H); 3.30-3.45 (m, 2H); 3.55-3.65 (m, 2H); 3.65-3.75 (m, 2H); 4.65 (s, 1.2H); 4.75 (s, 0.8H); 7.00 (t, 1H); 7.15-7.25 (m, 6H); 7.70 (d, 1H); 10.70 (broad s, 1H);11.30 (s, 1H). MS m/z: 422 (MH+), 247.

### 6c, 5-Chloro-3-{1-[4-(3,4-dihydro-2H-quinolin-1-yl)-4-oxobutan-1-yl]piperidin-4-yl}-1H-indole, hydrochloride

from 5-chloro-3-(piperidin-4-yl)-1*H*-indole and 4-chloro-1-(3,4-dihydro-2*H*-quinolin-1-yl)butan-1-one. Mp 158-162°C. ¹H NMR (DMSO-d₆): 1.85-1.95 (m, 2H); 1.95-2.20 (m, 6H); 2.60-2.75 (m, 4H); 2.95-3.15 (m, 5H); 3.55 (d, 2H); 3.70 (t, 2H); 7.05-7.25 (m, 6H); 7.40 (d, 1H); 7.75 (s, 1H); 10.45 (broad s, 1H); 11.15 (s, 1H). MS m/z: 436 (MH+), 303.

### Example 7

### 7, 5-Fluoro-3-{1-[4-(3,4-dihydro-1H-isoquinolin-2-yl)-4-oxobutan-1-yl]piperidin-4-yl}-1H- indole

A mixture of 5-fluoro-3-(piperidin-4-yl)-1*H*-indole (3.0 g), butanone (200 mL), tetrahydrofuran (200 mL), methanol (30 mL), potassium iodide (11.4 g) and triethylamine (7.6 mL) was heated until reflux temperature followed by the addition of a solution of 4-chloro-1-(3,4-dihydro-1*H*-isoquinolin-2-yl)butan-1-one (14.6 g) in butanone (50 mL). The mixture was boiled under reflux for 2 h, filtered hot and concentrated *in vacuo.* The residue was purified by flash chromatography on silicagel (eluent: ethyl acetate/ethanol/triethylamine 100:5:5) to give the crude product. The title compound was isolated as the free base from ethyl acetate as a white crystalline compound (0.9 g). Mp 146-148 °C. ¹H NMR (DMSO-d₆): 1.55-1.70 (m, 2H); 1.70-1.80 (m, 2H); 1.85-1.95 (m, 2H); 2.00 (q, 2H); 2.30 (q, 2H); 2.35-2.45 (m, 2H); 2.60-2.70 (m, 1H); 2.75 (t, 0.8H); 2.80-3.00 (m, 3.2H); 3.65 (t, 2H); 4.60 (s, 1.2H); 4.70 (s, 0.8H); 6.85-6.95 (m, 1H); 7.10-7.20 (m, 5H); 7.25 (d, 1H); 7.30-7.35 (m, 1H); 10.85 (s, 1H). MS m/z: 420 (MH+), 202.

### Example 8

### 8, 5-Chloro-3-{1-[4-(3, 4-dihydro-1H-isoquinolin-2-yl)-4-oxobutan-1-yl]piperidin-4-y}-1H-indole

A mixture of 5-fluoro-3-(piperidin-4-yl)-1*H*-indole (3.0 g), butanone (200 mL) and triethylamine (8.9 mL) was heated until reflux temperature followed by the addition of a solution of 4-chloro-1-(3,4-dihydro-1*H*-isoquinolin-2-yl)butan-1-one (15.2 g) in butanone (80 mL). The mixture was boiled under reflux for 6 h. The resulting mixture was filtered and concentrated *in vacuo.* The residue was purified by flash chromatography on silicagel (eluent: ethyl acetate/ethanol/triethylamine 100:4:4) to give the crude product. The title compound was isolated as the free base from acetone as a white crystalline compound (0.6 g). Mp 172-175 °C. ¹H NMR (DMSO-d₆): 1.55-1.65 (m, 2H); 1.65-1.75 (m, 2H); 1.90 (s, 2H); 2.00 (q, 2H); 2.30 (q, 2H); 2.40 (q, 2H); 2.65-2.80 (m, 1.8H); 2.80-3.00 (m, 3.2H); 3.70 (t, 2H); 4.60 (s, 1.2H); 4.70 (s, 0.8H); 7.05 (d, 1H); 7.10-7.25 (m, 5H); 7.35 (d, 1H); 7.55 (s, 1H); 11.00 (s, 1H). MS m/z: 436 (MH+), 202.

### Example 9

### 9a, 5-Fluoro-3-{1-[3-(3,4-dihydro-2H-quinolin-1-yl)propan-1-yl]piperidin-4-yl}-1H-indole, dihydrochloride

A suspension of lithium aluminium hydride (0.94 g) in tetrahydrofuran (40 mL) was stirred at 5 °C followed by the addition of concentrated sulphuric acid (1.2 g) in tetrahydrofuran (20 mL). The mixture was stirred at 7 °C for 60 min followed by the addition of 5-fluoro-3-{1-[3-(2-oxo-3,4-dihydro-2*H*-quinolin-1-yl)propan-1-yl]piperidin-4-yl}-1*H*-indole (2.0 g) in tetrahydrofuran (60 mL). The resulting mixture was stirred at 5 °C for 60 min followed by standard work up. The residue was purified by flash chromatography on silicagel (eluent: ethyl acetate) to give the crude product as a colourless oil. The title compound was isolated as the dihydrochloride salt from tetrahydrofuran as a white crystalline compound (1.0 g). Mp 230-236 °C. ¹H NMR (DMSO-d₆): 1.95 (t, 2H); 2.00-2.30 (m, 4H); 2.75 (t, 2H); 2.95-3.20 (m, 5H); 3.30 (t, 2H); 3.40 (t, 2H); 3.55 (d, 2H)6.20 (broad s, 1H); 6.70 (broad s, 1H); 6.95 (m, 2H); 7.00 (d, 1H); 7.10 (t, 1H); 7.20 (s, 1H); 7.30-7.40 (m, 1H); 7.50 (d, 1H); 10.95 (broad s, 1H); 11.05 (s, 1H). MS m/z: 392 (MH+), 259.

The following compounds were prepared in a similar manner

### 9b, 5-Fluoro-3{1-[4-(3,4-dihydro-2H-quinolin-1-yl)butan-1-yl]piperidin-4-yl}-1H-indole, dihydrochloride

from 5-fluoro-3-{1-[4-(2-oxo-3,4-dihydro-2*H*-quinolin-1-yl)butan-1-yl]piperidin-4-yl}-1*H*-indole. Mp 207-212 °C. ¹H NMR (DMSO-d₆): 1.65 (s, 2H); 1.80-1.90 (m, 2H); 1.95 (s, 2H); 2.05 (d, 2H); 2.20 (q, 2H); 2.65-2.80 (m, 2H); 2.95-3.25 (m, 4H); 3.15-3.25 (m, 1H); 3.35 (s, 4H); 3.55 (d, 2H); 4.65 (broad s); 5.55-6.95 (m, 3H); 7.00 (s, 1H); 7.10 (s, 1H); 7.20 (s, 1H); 7.30-7.40 (m, 1H); 7.55 (d, 1H); 11.75 (broad s, 1H); 11.05 (s, 1H). MS m/z: 406 (MH+), 274.

### 9c, 5-Fluoro-3-{1-[5-(3,4-dihydro-2H-quinolin-1-yl)pentan-1-yl]piperidin-4-yl}-1H-indole, dihydrochloride

from 5-fluoro-3-{1-[5-(2-oxo-3,4-dihydro-2*H*-quinolin-1-yl)pentan-1-yl]piperidin-4-yl}-1*H*-indole. Mp 155-158 °C. ¹H NMR (DMSO-d₆): 1.30-145 (m, 2H); 1.65 (s, 2H); 1.75-1.80 (m, 2H); 1.95 (s, 2H); 2.20 (q, 2H); 2.75 (s, 2H); 2.95-3.10 (m, 5H); 3.35 (s, 4H); 3.55 (d, 2H); 5.05 (broad s); 6.70-7.15 (m, 4H); 6.90 (t, 1H); 7.20 (s, 1H); 7.30-7.40 (m, 1H); 7.50 (d, 1H); 10.75 (broad s, 1H); 11.05 (s, 1H). MS m/z: 420 (MH+), 287.

### 9d, 5-Chloro-3-{1-[3-(3,4-dihydro-2H-quinolin-1-yl)propan-1-yl]piperidin-4-yl}-1H- indole, dihydrochloride

from 5-chloro-3-{1-[3-(2-oxo-3,4-dihydro-2*H*-quinolin-1-yl)propan-1-yl]piperidin-4-yl}-1*H*-indole. Mp 201-204 °C. ¹H NMR (DMSO-d₆): 1.95 (t, 2H); 2.00-2.25 (m, 6H); 2.75 (t, 2H); 3.00-3.20 (m, 5H); 3.30 (t, 2H); 3.40 (t, 2H); 3.55 (d, 2H); 6.40 (broad s); 6.65 (s, 1H); 6.85 (s, 1H); 6.95 (d, 1H); 7.00-7.10 (m, 2H); 7.20 (s, 1H); 7.40 (d, 1H); 7.75 (s, 1H); 10.85 (broad s, 1H); 11.20 (s, 1H). MS m/z: 408 (MH+), 275.

### 9e, 5-Chloro-3-{1-[4-(3,4-dihydro-2H-quinolin-1-yl)butan-1-yl]piperidin-4-yl}-1H-indole, dihydrochloride

from 5-chloro-3-{1-[4-(2-oxo-3,4-dihydro-2*H*-quinolin-1-yl)butan-1-yl]piperidin-4-yl}-1*H*-indole. Mp 140-145°C. ¹H NMR (DMSO-d₆): 1.65 (s, 2H); 1.80-1.90 (m, 2H); 1.95 (s, 2H); 2.00-2.25 (m, 4H); 2.75 (s, 2H); 2.95-3.25 (m, 5H); 3.35 (s, 4H); 3.55 (d, 2H); 6.75 (broad s, 1H); 6.90 (broad s, 1H); 7.00 (s, 1H); 7.05-7.15 (m, 2H); 7.20 (s, 1H); 7.40 (d, 1H); 7.80 (s, 1H); 10.70 (broad s, 1H); 11.20 (s, 1H). MS m/z: 422 (MH+), 289, 188.

### 9f, 5-Chloro-3-{1-[5-(3,4-dihydro-2H-quinolin-1-yl)pentan-1-yl]piperidin-4-yl}-1H-indole, dihydrochloride

from 5-chloro-3-{1-[5-(2-oxo-3,4-dihydro-2*H*-quinolin-1-yl)pentan-1-yl]piperidin-4-yl}-1*H*-indole. Mp 101-106 °C. ¹H NMR (DMSO-d₆): 1.30-1.45 (m, 2H); 1.65 (s, 2H); 1.70-1.85 (m, 2H); 1.95 (s, 2H); 2.00-2.25 (m, 4H); 2.75 (s, 2H); 2.95-3.25 (m, 5H); 3.35 (s, 4H); 3.55 (d, 2H); 6.80 (broad s, 1H); 6.90-7.15 (m, 4H); 7.20 (s, 1H); 7.35 (d, 1H); 7.75 (s, 1H); 10.70 (broad s, 1H); 11.20 (s, 1H). MS m/z: 436 (MH+), 303.

### 9g, 7-Chloro-3-{1-[4-(3,4-dihydro-2H-quinolin-1-yl)butan-1-yl]piperidin-4-yl}-1H-indole, dihydrochloride

from 7-chloro-3-{1-[4-(2-oxo-3,4-dihydro-2*H*-quinolin-1-yl)butan-1-yl]piperidin-4-yl}-1*H*-indole. Mp 214-219 °C. ¹H NMR (DMSO-d₆): 1.65 (s, 2H); 1.80-1.90 (m, 2H); 1.95 (s, 2H); 2.00-2.15 (m, 2H); 2.15-2.30 (m, 2H); 2.70 (s, 2H); 2.95-3.15 (m, 5H); 3.35 (s, 4H); 3.55 (d, 2H); 6.70 (broad s, 1H); 6.85 (broad s, 1H); 6.95-7.05 (m, 2H); 7.10 (s, 1H); 7.15-7.25 (m, 2H); 7.70 (d, 1H); 10.80 (broad s, 1H); 11.30 (s, 1H). MS m/z: 422 (MH+), 289, 188.

### 9h, 5-Fluoro-1-methyl-3-{1-[3-(3,4-dihydro-2H-quinolin-1-yl)propan-1-yl]piperidin-4-yl}-1H-indole, dihydrochloride

from 5-fluoro-1-methyl-3-{1-[3-(2-oxo-3,4-dihydro-2*H*-quinolin-1-yl)propan-1-yl]piperidin-4-yl}-1*H*-indole. Mp 202-206 °C. ¹H NMR (DMSO-d₆): 1.85-1.95 (m, 2H); 2.00-2.10 (m, 4H); 2.10-2.25 (m, 2H); 2.65-2.75 (m, 2H); 2.95-3.15 (m, 5H); 3.25-3.35 (m, 2H); 3.35-3.40 (m, 2H); 3.55 (d, 2H); 3.75 (s, 3H); 6.65 (broad s, 1H); 6.80 (broad s, 1H); 6.90-7.10 (m, 3H); 7.20 (s, 1H); 7.35-7.45 (m, 1H); 7.55 (d, 1H); 10.90 (broad s, 1H). MS m/z: 406 (MH+), 273.

### 9i, 5-Fluoro-1-methyl-3-{1-[4-(3,4-dihydro-2H-quinolin-1-yl)butan-1-yl]piperidin-4-yl}-1H-indole, oxalate

from 5-fluoro-1-methyl-3-{1-[4-(2-oxo-3,4-dihydro-2*H*-quinolin-1-yl)butan-1-yl]piperidin-4-yl}-1*H*-indole. Mp 123-125 °C. ¹H NMR (DMSO-d₆): 1.50-1.60 (m, 2H); 1.65-1.75 (m, 2H); 1.80-1.90 (m, 2H); 1.90-2.00 (m, 2H); 2.10 (d, 2H); 2.60-2.70 (m, 2H); 2.95-3.10 (m, 5H); 3.20-3.30 (m, 4H); 3.50 (d, 2H); 3.75 (s, 3H); 6.45 (t, 1H); 6.60 (d, 1H); 6.85 (d, 1H); 6.95-7.05 (m, 2H); 7.20 (s, 1H); 7.40-7.45 (m, 2H). MS m/z: 420 (MH+), 287.

### 9j, 5-Fluoro-3-{1-[4-(3,4-dihydro-2H-1,4-benzoxazin-4-yl)butan-1-yl]piperidin-4-yl}-1H-indole, dihydrochloride

from 5-fluoro-3-{1-[4-(3-oxo-3,4-dihydro-2*H*-1,4-benzoxazin-4-yl)butan-1-yl]piperidin-4-yl}-1*H*-indole. Mp 179-186 °C. ¹H NMR (DMSO-d₆): 1.55-1.65 (m, 2H); 1.75-1.90 (m, 2H); 2.00-2.10 (m, 2H); 2.15-2.25 (m, 2H); 2.95-3.25 (m, 5H); 3.25-3.40 (m, 4H); 3.55 (d, 2H); 4.15-4.25 (m, 2H); 6.55 (t, 1H); 6.65 (d, 1H); 6.70-6.80 (m, 2H); 6.90 (t, 1H); 7.20 (s, 1H); 7.30-7.40 (m, 1H); 7.55 (d, 1H); 10.80 (broad s, 1H); 11.05 (s, 1H). MS m/z: 408 (MH+), 273, 190.

### 9k, 5-Chloro-3-{1-[4-(3,4-dihydro-2H-1,4-benzoxazin-4-yl)butan-1-yl]piperidin-4-yl}-1H- indole, dihydrochloride

from 5-chloro-3-{1-[4-(3-oxo-3,4-dihydro-2*H*-1,4-benzoxazin-4-yl)butan-1-yl]piperidin-4-yl}-1*H*-indole. Mp 186-190°C. ¹H NMR (DMSO-d₆): 1.55-1.65 (m, 2H); 1.70-1.85 (m, 2H); 2.00-2.20 (m, 4H); 2.95-3.25 (m, 5H); 3.25-3.40 (m, 4H); 3.55 (d, 2H); 4.15-4.20 (m, 2H); 6.55 (t, 1H); 6.65 (d, 1H); 6.70-6.80 (m, 2H); 7.05 (d, 1H); 7.20 (s, 1H); 7.40 (d, 1H); 7.75 (s, 1H); 10.50 (broad s, 1H); 11.15 (s, 1H). MS m/z: 424 (MH+), 289, 190.

### 9l, 5-Fluoro-3-{1-[3-(3,4-dihydro-2H-quinolin-1-yl)propan-1-yl]-3,6-dihydro-2H-pyridin-4-yl}-1H-indole, dihydrochloride

from 5-fluoro-3-{1-[3-(2-oxo-3,4-dihydro-2*H*-quinolin-1-yl)propan-1-yl]-3,6-dihydro-2*H*-pyridin-4-yl}-1*H*-indole. Mp 220-223°C. ¹H NMR (DMSO-d₆): 1.85-2.00 (m, 2H); 2.05-2.10 (m, 2H); 2.70-2.80 (m, 4H); 2.90-3.00 (m, 1H); 3.15-3.30 (m, 2H); 3.30-3.35 (m, 2H); 3.40 (t, 2H); 3.55-3.65 (m, 1H); 3.70-3.80 (m, 1H); 4.00 (d, 1H); 6.10 (s, 1H); 6.70 (broad s, 1H); 6.90 (broad s, 1H); 6.95-7.05 (m, 2H); 7.05-7.10 (m, 1H); 7.40-7.45 (m, 1H); 7.55-7.65 (m, 2H); 11.10 (broad s, 1H); 11.60 (s, 1H). MS m/z: 390 (MH+), 203, 146.

### 9m, 5-Fluoro-3-{1-[4-(3,4-dihydro-2H-quinolin-1-yl)butan-1-yl]-3,6-dihydro-2H-pyridin-4-yl}-1H-indole, dihydrochloride

from 5-fluoro-3-{1-[4-(2-oxo-3,4-dihydro-2*H*-quinolin-1-yl)butan-1-yl]-3,6-dihydro-2*H*-pyridin-4-yl}-1H-indole. Mp 198-200 °C. ¹H NMR (DMSO-d₆): 1.60-1.75 (m, 2H); 1.80-1.90 (m, 2H); 1.95 (s, 2H); 2.70-2.80 (m, 4H); 2.85-3.00 (m, 1H); 3.15-3.30 (m, 4H); 3.30-3.40 (m, 2H); 3.55-3.65 (m, 1H); 3.70-3.80 (m, 1H); 3.95 (d, 1H); 6.10 (s, 1H); 6.80 (broad s, 1H); 6.90-7.20 (m, 3H); 7.00 (t, 1H); 7.40-7.45 (m, 1H); 7.55-7.65 (m, 2H); 10.95 (broad s, 1H); 11.55 (s, 1H). MS m/z: 404 (MH+), 271, 217.

### 9n, 5-Fluoro-3-{1-[5-(3, 4-dihydro-2H-quinolin-1-yl)pentan-1-yl]-3,6-dihydro-2H-pyridin-4-yl}-1H-indole, dihydrochloride

from 5-fluoro-3-{1-[5-(2-oxo-3,4-dihydro-2*H*-quinolin-1-yl)pentan-1-yl]-3,6-dihydro-2*H*-pyridin-4-yl}-1*H*-indole. Mp 167-169 °C. ¹H NMR (DMSO-d₆): 1.30-1.45 (m, 2H); 1.70 (s, 2H); 1.75-1.90 (m, 2H); 2.00 (s, 2H); 2.70-2.85 (m, 3H); 2.85-3.00 (m, 1H); 3.05-3.20 (m, 2H); 3.20-3.30 (m, 1H); 3.35 (s, 2H); 3.55-3.65 (m, 1H); 3.70-3.80 (m, 1H); 3.95 (d, 1H); 6.10 (s, 1H); 6.80-7.25 (m, 4H); 7.00 (t, 1H); 7.40-7.45 (m, 1H); 7.55-7.65 (m, 2H); 11.00 (s, broad s, 1H); 11.60 (s, 1H). MS m/z: 418 (MH+), 231, 188.

### Example 10

### 10a, 4-Fluoro-3-{1-[3-(3,4-dihydro-1H-isoquinolin-2-yl)-3-oxopropan-1-yl]piperidin-4-yl}-1H-indole

Polymer bound 3-[1-(4-fluoro-1*H*-indol-3-yl)piperidin-1-yl)propionic acid (0.1 g, 0.08 mmol) and dry dichloromethane (1 mL) were mixed in a reactor tube. The mixture was cooled to 0 °C and treated for 2 h with a 2 M solution of thionyl chloride (0.4 mL, 0.8 mmol) in dichloromethane. The resin was filtered off and washed with dry dichloromethane (3 × 1 mL), resuspended in dichloromethane (1 mL), and treated for 3 h at room temperature with 3,4-dihydro-1*H*-isoquinoline (0.05 g, 0.4 mmol). The resin was filtered off and washed with dichloromethane (3 × 1 mL), a 1:1 mixture of dichloromethane:triethylamine (3 × 1 mL) and dry dichloromethane (3 × 1 mL). The resin was treated for 1 h with 1 mL of a mixture of sodium methoxide (2 mL, 5 N sodium methoxide in methanol), methanol (50 mL) and tetrahydrofuran (50 mL). After filtration, the resin was washed with methanol (1 mL). The combined filtrates were loaded on a pre-conditioned ion exchange column (500 mg SCX column, commercially available from Analytical Instruments, part no. 1210-2040), washed with acetonitrile (1 mL) and methanol (1 mL). The product was eluted with 4 M ammonia in methanol. After evaporation of volatile solvents, the crude product was purified by preparative reversed phase HPLC chromatography. The resulting solution was subsequently loaded on a pre-conditioned ion exchange column washed with acetonitrile (1 mL) and methanol (1 mL). The product was eluted with 4 M ammonia in methanol. Evaporation of volatile solvents afforded the title compound as an yellow oil (5 mg, 12 µmol). LC/MS (m/z) 406 (MH+), RT = 3.61, purity: 66%.

The following compounds were prepared in a similar manner **(10b-10m)** or by the use of 3,4-dihydro-2*H*-quinoline **(10n-10z):**

### 10b, 4-Fluoro-3-{1-[4-(3,4-dihydro-1H-isoquinolin-2-yl)-4-oxobutan-1-yl]piperidin-4-yl}-1H-indole

LC/MS (m/z) 420 (MH+), RT = 3.69, purity: 93%

### 10c, 4-Fluoro-3-{1-[6-(3,4-dihydro-1H-isoquinolin-2-yl)-6-oxohexan-1-yl]piperidin-4-yl}-1H-indole

LC/MS (m/z) 448 (MH+), RT = 3.81, purity: 97%

### 10d, 4-Chloro-3-{1-[4-(3,4-dihydro-1H-isoquinolin-2-yl)-4-oxobutan-1-yl]piperidin-4-yl}-1H-indole

LC/MS (m/z) 436 (MH+), RT = 3.86, purity: 97%

### 10e, 4-Chloro-3-{1-{5-(3,4-dihydro-1H-isoquinolin-2-yl)-5-oxopentan-1-yl]piperidin-4-yl}-1H-indole

LC/MS (m/z) 450 (MH+), RT = 3.87, purity: 81%

### 10f, 4-Chloro-3-{1-[6-(3,4-dihydro-1H-isoquinolin-2-yl)-6-oxohexan-1-yl]piperidin-4-yl}-1H-indole

LC/MS (m/z) 464 (MH+), RT = 3.97, purity: 86%

### 10g, 5-Fluoro-3-{1-[5-(3,4-dihydro-1H-isoquinolin-2-yl)-5-oxopentan-1-yl]piperidin-4-yl}-1H-indole

LC/MS (m/z) 434 (MH+), RT = 3.67, purity: 93%

### 10h, 5-Fluoro-3-{1-[6-(3,4-dihydro-1H-isoquinolin-2-yl)-6-oxohexan-1-yl]piperidin-4-yl}-1H-indole

LC/MS (m/z) 448 (MH+), RT = 3.79, purity: 89%

### 10i, 6-Chloro-3-{1-[3-(3,4-dihydro-1H-isoquinolin-2-yl)-3-oxopropan-1-yl]piperidin-4-yl}-1H-indole

LC/MS (m/z) 422 (MH+), RT = 3.80, purity: 85%

### 10j, 6-Chloro-3-{1-[6-(3,4-dihydro-1H-isoquinolin-2-yl)-6-oxohexan-1-yl]piperidin-4-yl}-1H-indole

LC/MS (m/z) 464 (MH+), RT = 3.98, purity: 87%

### 10k, 7-Chloro-3-{1-[4-(3,4-dihydro-1H-isoquinolin-2-yl)-4-oxobutan-1-yl]piperidin-4-yl}-1H-indole

LC/MS (m/z) 436 (MH+), RT = 3.85, purity: 98%

### 10l, 7-Chloro-3-{1-[5-(3,4-dihydro-1H-isoquinolin-2-yl)-5-oxopentan-1-yl]piperidin-4-yl}-1H-indole

LC/MS (m/z) 450 (MH+), RT = 3.85, purity: 96%

### 10m, 7-Chloro-3-{1-[6-(3,4-dihydro-1H-isoquinolin-2-yl)-6-oxohexan-1-yl]piperidin-4-yl}-1H-indole

LC/MS (m/z) 464 (MH+), RT = 3.96, purity: 97%

### 10n, 4-Fluoro-3-{1-[3-(3,4-dihydro-2H-quinolin-1-yl)-3-oxopropan-1-yl]piperidin-4-yl}-1H-indole

LC/MS (m/z) 406 (MH+), RT = 3.67, purity: 82%

### 10o, 4-Fluoro-3-{1-[4-(3,4-dihydro-2H-quinolin-1-yl)-4-oxobutan-1-yl]piperidin-4-yl}-1H- indole

LC/MS (m/z) 420 (MH+), RT = 3.78, purity: 84%

### 10p, 4-Chloro-3-{1-[3-(3,4-dihydro-2H-quinolin-1-yl)-3-oxopropan-1-y]piperidin-4-yl}-1H-indole

LC/MS (m/z) 422 (MH+), RT = 3.85, purity: 97%

### 10q, 4-Chloro-3-{1-[4-(3,4-dihydro-2H-quinolin-1-yl)-4-oxobutan-1-yl]piperidin-4-yl}-1H- indole

LC/MS (m/z) 436 (MH+), RT = 3.97, purity: 92%

### 10r, 5-Fluoro-3-{1-[3-(3,4-dihydro-2H-quinolin-1-yl)-3-oxopropan-1-yl]piperidin-4-yl}-1H-indole

LC/MS (m/z) 406 (MH+), RT = 3.63, purity: 97%

### 10s, 5-Fluoro-3-{1-[4-(3,4-dihydro-2H-quinolin-1-yl)-4-oxobutan-1-yl]piperidin-4-yl}-1H- indole

LC/MS (m/z) 420 (MH+), RT = 3.73, purity: 96%

### 10t, 5-Fluoro-3-{1-[5-(3,4-dihydro-2H-quinolin-1-yl)-5-oxopentan-1-yl]piperidin-4-yl}-1H-indole

LC/MS (m/z) 434 (MH+), RT = 3.76, purity: 97%

### 10u, 5-Fluoro-3-{1-[6-(3,4-dihydro-2H-quinolin-1-yl)-6-oxohexan-1-yl]piperidin-4-yl}-1H-indole

LC/MS (m/z) 448 (MH+), RT = 3.88, purity: 97%

### 10v, 6-Chloro-3-{1-[3-(3,4-dihydro-2H-quinolin-1-yl)-3-oxopropan-1-yl]piperidin-4-yl}-1H-indole

LC/MS (m/z) 422 (MH+), RT = 3.88, purity: 90%

### 10w, 6-Chloro-3-{1-[6-(3,4-dihydro-2H-quinolin-1-yl)-6-oxohexan-1-yl]piperidin-4-yl}-1H-indole

LC/MS (m/z) 464 (MH+), RT = 4.09, purity: 96%

### 10x, 7-Chloro-3-{1-[4-(3,4-dihydro-2H-quinolin-1-yl)-4-oxobutan-1-yl]piperidin-4-yl}-1H-indole

LC/MS (m/z) 436 (MH+), RT = 3.91, purity: 98%

### 10y, 7-Chloro-3-{1-[5-(3,4-dihydro-2H-quinolin-1-yl)-5-oxopentan-1-yl]piperidin-4-yl}-1H-indole

LC/MS (m/z) 450 (MH+), RT = 3.93, purity: 96%

### 10z, 7-Chloro-3-{1-[6-(3,4-dihydro-2H-quinolin-1-yl)-6-oxohexan-1-yl]piperidin-4-yl}-1H-indole

LC/MS (m/z) 464 (MH+), RT = 4.05, purity: 97%

### Example 11

### 11a, 5-Fluoro-3-{1-[4-(3,4-dihydro-1H-isoquinolin-2-yl)butan-1-yl]piperidin-4-yl}-1H-indole, dioxalate

A mixture of 5-fluoro-3-(piperidin-4-yl)-1*H*-indole (5.0 g), triethylamine (6.35 mL) and tetrahydrofuran (500 mL) was cooled to 7 °C and subsequently added a mixture of succinic anhydride (2.5 g) in tetrahydrofuran (50 mL). The mixture was stirred at 8-10 °C for 2 h, and the solvent was removed *in vacuo.* The residue was dissolved in ethyl acetate, and the organic phase was washed with cold 2N aqueous hydrochloride solution and brine. The organic phase was dried (MgSO₄), filtered and concentrated *in vacuo* (6.4 g). The residue (1.5 g) and 3,4-dihydro-1*H*-isoquinoline (0.63 g) was dissolved in a mixture of acetonitril (25 mL) and dimethyl formamide (10 mL), and the resulting mixture was cooled (5 °C) and subsequently added 1,3-dicyclohexylcarbodiimide (1.0 g). The mixture was stirred at room temperature for 16 h, filtered and poured into brine. The aqueous phase was extracted with ethyl acetate and tetrahydrofuran, and the combined organic phase was washed with brine, dried (MgSO₄) and concentrated *in vacuo.* The residue was purified by flash chromatography on silicagel (eluent: ethyl acetate) to give a white solid (1.0 g), which subsequently was added to a mixture of alane in tetrahydrofuran (100 mL) at 5-10 °C. The alane was prepared from lithium aluminium hydride (0.55 g) and concentrated sulphuric acid (0.72 g). The mixture was quenched by the addition of water (1 mL), 15 % aqueous sodium hydroxide solution (0.5 mL) and water (2.5 mL), and the resulting mixture was dried (MgSO₄), filtered and concentrated *in vacuo.* The title compounds was crystallised from acetone as the dioxalate salt (0.8 g). Mp 105-111 °C. ¹H NMR (DMSO-d₆): 1.75 (s, 4H); 1.85-2.05 (m, 2H); 2.10 (d, 2H); 2.90-3.20 (m, 9H); 3.25 (t, 2H); 3.50 (d, 2H); 4.15 (s, 2H); 6.85-6.95 (m, 1H); 7.10-7.25 (m, 5H); 7.30-7.45 (m, 2H); 11.05 (s, 1H). MS m/z: 406 (MH+), 273, 188.

The following compound was prepared in a similar manner

### 11b, 5-Fluoro-3-{1-[4-(6,7-dimethoxy-3,4-dihydro-1H-isoquinolin-2-yl)butan-1-yl]peridin-4-yl}-1H-indole, dioxalate

from 5-fluoro-3-(piperidin-4-yl)-1*H*-indole and 6,7-dimethoxy-3,4-dihydro-1*H*-isoquinoline. Mp 98-105 °C. ¹H NMR (DMSO-d₆): 1.75 (s, 4H); 1.85-2.00 (m, 2H); 2.10 (d, 2H); 2.90-3.15 (m, 9H); 3.30 (s, 2H); 3.50 (d, 2H); 3.75 (d, 6H); 4.10 (s, 2H); 6.75 (s, 1H); 6.80 (s, 1H); 6.90-6.95 (m, 1H); 7.20 (s, 1H); 7.30-7.45 (m, 2H); 11.05 (s, 1H). MS m/z: 466 (MH+), 273, 248.

### Pharmacological Testing

The compounds of the invention were tested in well-recognised and reliable tests. The tests were as follows:

### Inhibition of the binding of [³H]YM-09151-2 to human dopamine D₄ receptors

By this method, the inhibition by drugs of the binding of [³H]YM-09151-2 (0.06 nM) to membranes of human cloned dopamine D_{4.2} receptors expressed in CHO-cells is determined *in vitro.* Method modified from NEN Life Science Products, Inc., technical data certificate PC2533-10/96.

In table 1 below, the test results are shown:

**Table 1:Binding Data (IC₅₀ values in nM or % inhibition of binding at 50nM)(nt. means not tested)**

| **Comp. No.** | **D₄-bind.** | **Comp. No.** | **D₄-bind.** | **Comp. No.** | **D₄-bind.** |
|---|---|---|---|---|---|
| **1a** | 92% | **3a** | 0.71 | **10e** | 25% |
| **1b** | 97% | **3b** | 5.0 | **10f** | 17% |
| **1c** | 95% | **3c** | 15 | **10g** | 65% |
| **2a** | 0.58 | **4** | 4.8 | **10h** | 50% |
| **2b** | 12 | **9l** | 0.51 | **10i** | 94% |
| **2c** | 0.69 | **9m** | 17 | **10j** | 70% |
| **2d** | 8.0 | **9n** | 53 | **10k** | 95% |
| **2e** | 12 | **10a** | 93% | **10l** | 82% |
| **2f** | 78% | **10b** | 81% | **10m** | 69% |
| **2g** | 7.5 | **10c** | 21% | | |
| **2h** | 10 | **10d** | 86% | | |

The compounds of the invention have been found potently to inhibit the binding of tritiated YM-09151-2 to dopamine D₄ receptors.

The compounds have also been tested in a functional assay described by Gazi et al. in British Journal of Pharmacology 1999, 128, 613-620. In this test, the compounds were shown to be partial agonists or antagonists at the dopamine D₄ receptors.

The compounds of the invention have also been tested in the following tests:

### Inhibition of the binding of [³H]Spiperone to D₂ receptors

The compounds of the invention were tested with respect to affinity for the dopamine D₂ receptor by determining their ability to inhibit the binding of [³H]spiperone to D₂ receptors by the method of Hyttel et al. J. Neurochem. 1985, 44, 1615.

### Inhibition of the binding of [³H]Spiperone to human D₃ receptors

By this method, the inhibition by drugs of the binding [³H]Spiperone (0.3 nM) to membranes of human cloned dopamine D₃ receptors expressed in CHO-cells is determined *in vitro.* Method modified from MacKenzie et al. Eur. J. Pharm.-Mol. Pharm. Sec. 1994, 266, 79-85.

### Inhibition of the uptake of [³H]Serotonin into whole rat brain synaptosomes

The compounds were tested with respect to their 5-HT reuptake inhibiting effect by measuring their ability to inhibit the uptake of [³H]serotonin into whole rat brain synaptosomes *in vitro.* The assay was performed as described by Hyttel Psychopharmacology 1978, 60, 13.

### Inhibition of the binding of [³H]Ketanserin to 5-HT_{2A} receptors

The compounds were tested with respect to their affinity for 5-HT_{2A} receptors by determining their ability to inhibit the binding of [³H]Ketanserin (0.50 nM) to membranes from rat brain (cortex) *in vitro.* Method described in Sánchez et al. Drug Dev. Res. 1991, 22, 239-250.

### 5-HT_{2c} receptor efficacy as determined by fluorometry

The compounds were tested with respect to their efficacy on 5-HT_{2C} receptor-expressing CHO cells as determined by fluorometric imaging plate reader (FLIPR) analysis. This assay was carried out according to Molecular Devices Inc. instructions for their FLIPR Calcium Assay Kit and as modified from Porter et al. British Journal of Pharmacology 1999, 128:13.

The compounds were found to have no substantial or only weak affinity for the dopamine D₂ receptor.

Many of the compounds have been found to inhibit the binding of [³H]Spiperone to the dopamine D₃ receptor, some of the compounds have been found to inhibit serotonin reuptake and some of the compounds have been found to be 5-HT_{2A} receptor ligands and/or 5-HT_{2C} receptor ligands.

As mentioned above, the compounds of the invention have a good aqueous solubility as compared to related compounds disclosed in WO 98/28293. Accordingly, the compounds are expected to have improved bioavailability.

Thus, the compounds of the invention are considered useful in the treatment of positive and negative symptoms of schizophrenia, other psychoses, anxiety disorders, such as generalised anxiety disorder, panic disorder, and obsessive compulsive disorder, depression, side effects induced by conventional antipsychotic agents, migraine, ADHD and in the improvement of sleep. In particular, the compounds of the invention are considered useful in the treatment of positive and negative symptoms of schizophrenia without inducing extrapyramidal side effects.

### Formulation Examples

The pharmaceutical formulations of the invention may be prepared by conventional methods in the art.

For example: Tablets may be prepared by mixing the active ingredient with ordinary adjuvants and/or diluents and subsequently compressing the mixture in a conventional tabletting machine. Examples of adjuvants or diluents comprise: corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.
Solutions for injections may be prepared by dissolving the active ingredient and possible additives in a part of the solvent for injection, preferably sterile water, adjusting the solution to desired volume, sterilising the solution and filling it in suitable ampules or vials. Any suitable additive conventionally used in the art may be added, such as tonicity agents, preservatives, antioxidants, etc.
Typical examples of recipes for the formulation of the invention are as follows:
1) Tablets containing 5.0 mg of a compound of the invention calculated as the free base:

| | |
|---|---|
| Compound | 5.0 mg |
| Lactose | 60 mg |
| Maize starch | 30 mg |
| Hydroxypropylcellulose | 2.4 mg |
| Microcrystalline cellulose | 19.2 mg |
| Croscarmellose Sodium Type A | 2.4 mg |
| Magnesium stearate | 0.84 mg |

2) Tablets containing 0.5 mg of a compound of the invention calculated as the free base:

| | |
|---|---|
| Compound | 0.5 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Compound | 25 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 ml |
| Flavour | 0.05 mg |
| Saccharin sodium | 0.5 mg |
| Water | ad 1 ml |

4) Solution for injection containing per millilitre:

| | |
|---|---|
| Compound | 0.5 mg |
| Sorbitol | 5.1 mg |
| Acetic Acid | 0.05 mg |
| Saccharin sodium | 0.5 mg |
| Water | ad 1 ml |

## Claims

1. A substituted indole derivative of formula I 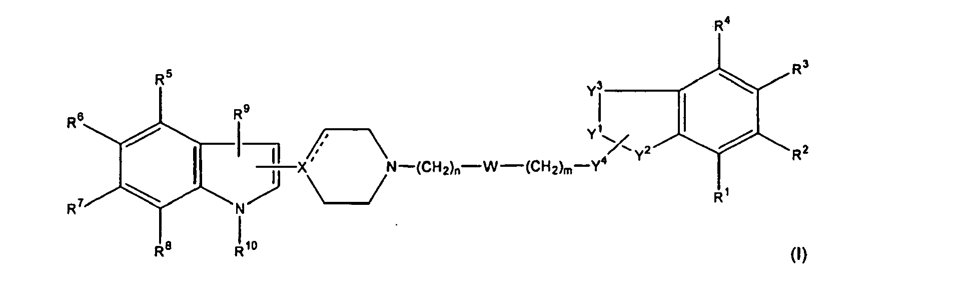 wherein one of Y¹ and Y² is N, which is bound to Y⁴, and the other of Y¹ and Y² is CH₂ and Y⁴ is CO, CS, SO or SO₂; or
Y³ is Z-CH₂, CH₂-Z or CH₂CH₂, and Z is O or S; provided that when Y¹ is N, Y³ may not Z-CH₂;
W is a bond or n O, S, CO, CS, SO or SO₂ group;
n is 0-5, m is 0-5 and m + n is 1 to 10; provided that when W is O or S, then n ≥ 2 and m ≥ 1; when W is CO, CS, SO or SO₂, then n ≥ 1 and m ≥ 1;
X is C, CH or N; provided that when X is C, the dotted line indicates a bond, and when X is N or CH, the dotted line is not a bond;
R¹ -R⁹ are independently selected from hydrogen, halogen, cyano, nitro, amino, hydroxy, C₁₋₆-alkyl-amino, di-C₁₋₆-alkyl-amino, C₁₋₆-alkyl, C₂₋₆-alkenyl, C₂₋₆-alkynyl, C₁₋₆ alkoxy, C₁₋₆-alkylthio, C₁₋₆-alkyl substituted with hydroxy or thiol, C₃₋₈-cycloalkyl, C₃₋₈-cycloalkyl-C₁₋₆-alkyl, acyl, thioacyl, aryl, trifluoromethyl, trifluoromethylsulfonyl, and C₁₋₆ alkylsulfonyl;
R¹⁰ is hydrogen, C₁₋₆-alkyl, C₂₋₆-alkenyl, C₂₋₆-alkynyl, C₁₋₆-alkyl substituted with hydroxy or thiol, C₃₋₈-cycloalkyl, C₃₋₈-cycloalkyl-C₁₋₆-alkyl, aryl, aryl-C₁₋₆-alkyl, acyl, thioacyl, C₁₋₆-alkylsulfonyl, trifluoromethylsulfonyl or arylsulfonyl, or a pharmaceutically acceptable acid addition salt thereof.

2. A compound according to claim 1 wherein the indole is bound to X via position 3 of the indole.

3. A compound according to claims 1 to 2 wherein one of Y¹ and Y² is N, which is bound to Y⁴, and the other of Y¹ and Y² is CH₂ and Y⁴ is CO.

4. A compound according to claim 3 wherein Y¹ is a nitrogen bound to Y⁴, Y² is CH₂ and Y⁴ is CO.

5. A compound according to claim 3 wherein Y² is a nitrogen atom bound to Y⁴, Y¹ is CH₂ and Y⁴ is CO.

6. A compound according to claims 1 to 5 wherein Y³ is CH₂CH₂ or CH₂Z.

7. A compound according to claims 1 to 6 wherein X is C.

8. A compound according to claims 1 to 6 wherein X is N.

9. A compound according to claims 1 to 6 wherein X is CH.

10. A compound according to claims 1 to 9 wherein R¹ -R⁹ are independently selected from hydrogen, halogen, cyano, nitro, amino, C₁₋₆-alkylamino, di-C₁₋₆-alkylamino, C₁₋₆-alkyl, C₃₋₈-cycloalkyl and trifluoromethyl, and R¹⁰ is hydrogen, C₁₋₆-alkyl or acyl, or a pharmaceutically acceptable acid addition salt thereof.

11. A compound according to claims 1 to 10 wherein W is a bond and n+m is 1 to 6.

12. A compound according to claim 11 wherein n + m is 3 to 6.

13. A compound according to claim 1 which is selected from:
5-Fluoro-3-{1-[3-(3,4-dihydro-1H-isoquinolin-2-yl)-3-oxopropan-1-yl]piperidin-4-y1}-1H-indole;
7-Chloro-3-{1-[3-(3,4-dihydro-1H-isoquinolin-2-yl)-3-oxopropan-1-yl]piperidin-4-y1}-1H-indole;
5-Chloro-3-{1-[4-(3,4-dihydro-2H-quinolin-1-yl)-4-oxobutan-1-yl]piperidin4-yl}-1H-indole;
5-Fluoro-3-{1-[4-(3,4-dihydro-1H-isoquinolin-2-yl)-4-oxobutan-1-yl]piperidin-4-yl}-1H-indole;
5-Chloro-3-{1-[4-(3,4-dihydro-1H-isoquinolin-2-yl)-4-oxobutan-1-yl]piperidin-4-yl}-1H-indole;
4-Fluoro-3- {1-[3-(3,4-dihydro-1H-isoquinolin-2-yl)-3-oxopropan-1-yl]piperidin-4-yl}-1H-indole;
4-Fluoro-3-{1-[4-(3,4-dihydro-1H-isoquinolin-2-yl)-4-oxobutan-1-yl]piperidin-4-yl}-1H-indole;
4-Fluoro-3-{1-[6-(3,4-dihydro-1 H-isoquinolin-2-yl)-6-oxohexan-1-yl]piperidin-4-yl}-1H-indole;
4-Chloro-3-{1-[4-(3,4-dihydro-1H-isoquinolin-2-yl)-4-oxobutan-1-yl]piperidin-4-yl}-1H-indole;
4-Chloro-3-1-[5-(3,4-dihydro-1H-isoquinolin-2-yl)-5-oxopentan-1-yl]piperidin-4-yl}-1H-indole;
4-Chloro-3 - {1-[6-(3,4-dihydro-1H-isoquinolin-2-yl)-6-oxohexan-1-yl]piperidin-4-yl}-1H-indole;
5-Fluoro-3-{1-[5-(3,4-dihydro-1H-isoquinolin-2-yl)-5-oxopentan-1-yl]piperidin-4-yl}-1H-indole;
5-Fluoro-3-{1-[6-(3,4-dihydro-1H-isoquinolin-2-yl)-6-oxohexan-1-yl]piperidin-4-yl}-1 H-indole:
6-Chloro-3-{1-[3-(3,4-dihydro-1H-isoquinolin-2-yl)-3-oxopropan-1-yl]piperidin-4-yl}-1H-indole;
6-Chloro-3-{ 1-[6-(3,4-dihydro-1H-isoquinolin-2-yl)-6-oxohexan-1-yl]piperidin-4-yl}-1H-indole;
7-Chloro-3 - {1-[4-(3,4-dihydro-1H-isoquinolin-2-yl)-4-oxobutan-1-yl]piperidin-4-yl}-1 H-indole;
7-Chloro-3-{1-[5-(3,4-dihydro-1H-isoquinolin-2-yl)-5-oxopentan-1-yl]piperidin-4-yl}-1H-indole;
7-Chloro-3-{1-[6-(3,4-dihydro-1H-isoquinolin-2-yl)-6-oxohexan-1-yl]piperidin-4-yl}-1H-indole;
4-Fluoro-3-{1-[3-(3,4-dihydro-2H-quinolin-1-yl)-3-oxopropan-1-yl]piperidin-4-yl}-1H-indole;
4-Fluoro-3-{1-[4-(3,4-dihydro-2H-quinolin-1-yl)-4-oxobutan-1-yl]piperidin-4-yl}-1H-indole;
4-Chloro-3-{1-[3-(3,4-dihydro-2H-quinolin-1-yl)-3-oxopropan-1-yl]piperidin-4-yl}-1H-indole;
4-Chloro-3-{1-[4-(3,4-dihydro-2H-quinolin-1-yl)-4-oxobutan-1-yl]piperidin-4-yl}-1H-indole;
5-Fluoro-3-{1-[3-(3,4-dihydro-2H-quinolin-1-yl)-3-oxopropan-1-yl]piperidin-4-yl}-1H-indole;
5-Fluoro-3-{1-[4-(3,4-dihydro-2H-quinolin-1-yl)-4-oxobutan-1-yl]piperidin-4-yl}-1H-indole;
5-Fluoro-3-{1-[5-(3,4-dihydro-2H-quinolin-1-yl)-5-oxopentan-1-yl]piperidin-4-yl}-1H-indole;
5-Fluoro-3-{1-[6-(3,4-dihydro-2H-quinolin-1-yl)-6-oxohexan-1-yl]piperidin-4-yl}-1H-indole;
6-Chloro-3-{1-[3-(3,4-dihydro-2H-quinolin-1-yl)-3-oxopropan-1-yl]piperidin-4-yl}-1 H-indole;
6-Chloro-3-{1-[6-(3,4-dihydro-2H-quinolin-1-yl)-6-oxohexan-1-yl]piperidin-4-yl}-1H-indole;
7-Chloro-3-{1-[4-(3,4-dihydro-2H-quinolin-1-yl)-4-oxobutan-1-yl]piperidin-4-yl}-1H-indole;
7-Chloro-3-{1-[5-(3,4-dihydro-2H-quinolin-1-yl)-5-oxopentan-1-yl]piperidin-4-yl}-1H-indole; and
7-Chloro-3-{1-[6-(3,4-dihydro-2H-quinolin-1-yl)-6-oxohexan-1-yl]piperidin-4-yl}-1H-indole;
or pharmaceutically acceptable salts thereof.

14. A pharmaceutical composition **characterised in that** it comprises a compound of any of claims 1 to 13 in a therapeutically effective amount together with one or more pharmaceutically acceptable carriers or diluents.

15. Use of a compound of any of Claims 1 to 13 for the manufacture of a medicament useful in the treatment of positive and negative symptoms of schizophrenia, other psychoses, anxiety disorders, such as generalised anxiety disorder, panic disorder and obsessive compulsive disorder, depression, aggression, side effects induced by conventional antipsychotic agents, migraine, cognitive disorders, ADHD and in the improvement of sleep.

## Patentansprüche

1. Substituiertes Indol-Derivat der Formel (I): 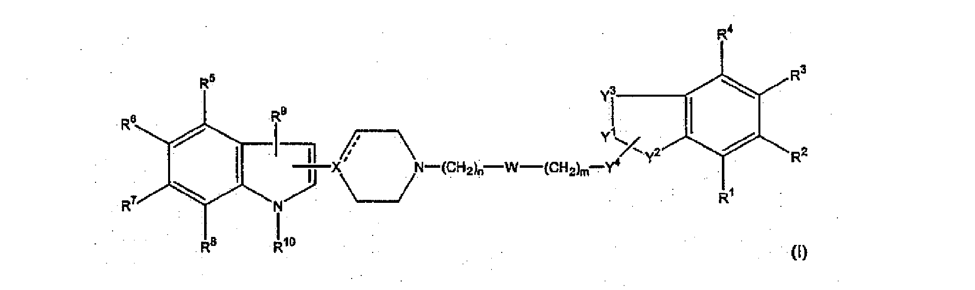 worin
ein Vertreter aus Y¹ und Y² N ist, das an Y⁴ gebunden ist, und der andere Vertreter aus Y¹ und Y² CH₂ ist und Y⁴ CO, CS, SO oder SO₂ ist;
Y³ Z-CH₂, CH₂-Z oder CH₂CH₂ ist und Z O oder S ist; mit der Massgabe, dass Y³ nicht Z-CH₂ sein kann, wenn Y¹ N ist;
W eine Bindung oder eine O-, S-, CO-, CS-, SO- oder SO₂-Gruppe ist;
n 0-5 ist, m 0-5 ist und m + n 1-10 ist; mit der Massgabe, dass dann, wenn W 0 oder S ist, n ≥ 2 und m ≥ 1 ist; und dann, wenn W CO, CS, SO oder SO₂ ist, n ≥ 1 und m ≥ 1 ist;
X C, CH oder N ist; mit der Massgabe, dass die gestrichelte Linie eine Bindung anzeigt, wenn X C ist,
und die gestrichelte Linie keine Bindung ist, wenn X N oder CH ist;
R¹-R⁹ unabhängig ausgewählt sind aus Wasserstoff, Halogen, Cyano, Nitro, Amino, Hydroxy, C₁₋₆-Alkylamino, Di-C₁₋₆-alkylamino, C₁₋₆-Alkyl, C₂₋₆-Alkenyl, C₂₋₆-Alkinyl, C₁₋₆-Alkoxy, C₁₋₆-Alkylthio, C₁₋₆-Alkyl, das mit Hydroxy oder Thiol substituiert ist, C₃₋₈-Cycloalkyl, C₃₋₈-Cycloalkyl-C₁₋₆-alkyl, Acyl, Thioacyl, Aryl, Trifluormethyl, Trifluormethylsulfonyl und C₁₋₆-Alkylsulfonyl;
R¹⁰ Wasserstoff, C₁₋₆-Alkyl, C₂₋₆-Alkenyl, C₂₋₆-Alkinyl, C₁₋₆-Alkyl, das mit Hydroxy oder Thiol substituiert ist, C₃₋₈-Cycloalkyl, C₃₋₈-Cycloalkyl-C₁₋₆-alkyl, Aryl, Aryl-C₁₋₆-alkyl, Acyl, Thioacyl, C₁₋₆-Alkylsulfonyl, Trifluormethylsulfonyl oder Arylsulfonyl ist, oder ein pharmazeutisch akzeptables Säureadditionssalz davon.

2. Verbindung gemäss Anspruch 1, worin das Indol an X über Position 3 des Indols gebunden ist.

3. Verbindung gemäss Ansprüchen 1 bis 2, worin ein Vertreter aus Y¹ und Y² N ist, das an Y⁴ gebunden ist, und der andere Vertreter aus Y¹ und Y² CH₂ ist und Y⁴ CO ist.

4. Verbindung gemäss Anspruch 3, worin Y¹ ein Stickstoffatom ist, das an Y⁴ gebunden ist, Y² CH₂ ist und Y⁴ CO ist.

5. Verbindung gemäss Anspruch 3, worin Y² ein Stickstoffatom ist, das an Y⁴ gebunden ist, Y¹ CH₂ ist und Y⁴ CO ist.

6. Verbindung gemäss Ansprüchen 1 bis 5, worin Y³ CH₂CH₂ oder CH₂Z ist.

7. Verbindung gemäss Ansprüchen 1 bis 6, worin X C ist.

8. Verbindung gemäss Ansprüchen 1 bis 6, worin X N ist.

9. Verbindung gemäss Ansprüchen 1 bis 6, worin X CH ist.

10. Verbindung gemäss Ansprüchen 1 bis 9, worin R¹-R⁹ unabhängig ausgewählt sind aus Wasserstoff, Halogen, Cyano, Nitro, Amino, C₁₋₆-Alkylamino, Di-C₁₋₆-alkylamino, C₁₋₆-Alkyl, C₃₋₈-Cycloalkyl und Trifluormethyl und R¹⁰ Wasserstoff, C₁₋₆-Alkyl oder Acyl ist, oder ein pharmazeutisch akzeptables Säureadditionssalz davon.

11. Verbindung gemäss Ansprüchen 1 bis 10, worin W eine Bindung ist und n+m 1 bis 6 ist.

12. Verbindung gemäss Anspruch 11, worin n+m 3 bis 6 ist.

13. Verbindung gemäss Anspruch 1, die ausgewählt ist aus:
5-Fluor-3-{1-[3-(3,4-dihydro-1H-isochinolin-2-yl)3-oxopropan-1-yl]piperidin-4-yl}-1H-indol;
7-Chlor-3-{1-[3-(3,4-dihydro-1H-isochinolin-2-yl)-3-oxopropan-1-yl]piperidin-4-yl}-1H-indol;
5-Chlor-3-{1-[4-(3,4-dihydro-2H-chinolin-1-yl)-4-oxobutan-1-yl]piperidin-4-yl}-1H-indol;
5-Fluor-3-{1-[4-(3,4-dihydro-1H-isochinolin-2-yl)-4-oxobutan-1-yl]piperidin-4-yl}-1H-indol;
5-Chlor-3-{1-[4-(3,4-dihydro-1H-isochinolin-2-yl)-4-oxobutan-1-yl]piperidin-4-yl}-1H-indol;
4-Fluor-3-{1-[3-(3,4-dihydro-1H-isochinolin-2-yl)-3-oxopropan-l-yl]piperidin-4-yl}-1H-indol;
4-Fluor-3-{1-[4-(3,4-dihydro-1H-isochinolin-2-yl)-4-oxobutan-1-yl]piperidin-4-yl}-1H-indol;
4-Fluor-3-{1-[6-(3,4-dihydro-1H-isochinolin-2-yl)-6-oxohexan-1-yl]piperidin-4-yl}-1H-indol;
4-Chlor-3-{1-[4-(3,4-dihydro-1H-isochinolin-2-yl)-4-oxobutan-1-yl]piperidin-4-yl}-1H-indol;
4-Chlor-3-{1-[5-(3,4-dihydro-1H-isochinolin-2-yl)-5-oxopentan-1-yl]piperidin-4-yl}-1H-indol;
4-Chlor-3-{1-[6-(3,4-dihydro-1H-isochinolin-2-yl)-5-oxohexan-1-yl]piperidin-4-yl}-1H-indol;
5-Fluor-3-{1-[5-(3,4-dihydro-1H-isochinolin-2-yl)-5-oxopentan-1-yl]piperidin-4-yl}1H-indol;
5-Fluor-3-{1-[6-(3,4-dihydro-1H-isochinolin-2-yl)-5-oxohexan-1-yl]piperidin-4-yl}-1H-indol;
6-Chlor-3-{1-[3-(3,4-dihydro-1H-isochinolin-2-yl)-3-oxopropan-1-yl]piperidin-4-yl]-1H-indol;
6-Chlor-3-{1-[6-(3,4-dihydro-1H-isochinolin-2-yl)-6-oxohexan-1-yl]piperidin-4-yl}-1H-indol;
7-Chlor-3-{1-[4-(3,4-dihydro-1H-isochinolin-2-yl)-4-oxobutan-1-y1]piperidin-4-y1}-2H-indol;
7-Chlor-3-{1-[5-(3,4-dihydro-1H-isochinolin-2-yl)-5-oxopentan-1-yl]piperidin-4-yl}-1H-indol;
7-Chlor-3-{1-[5-(3,4-dihydro-1H-isochinolin-2-yl)-6-oxohexan-1-yl]piperidin-4-yl}-1H-indol;
4-Fluor-3-{1-[3-(3,4-dihydro-2H-chinolin-1-yl)-3-oxopropan-1-yl]piperidin-4-yl}-1H-indol;
4-Fluor-3-{1-[4-(3,4-dihydro-2-chinolin-1-yl)-4-oxobutan-1-yl]piperidin-4-y1}-1H-indol;
4-Chlor-3-{1-[3-(3,4-dihydro-2H-chinolin-1-yl)-3-oxopropan-1-yl]piperidin-d-yl}-1H-indol;
4-Chlor-3-{1-[4-(3,4-dihydro-2H-chinolin-1-yl)-4-oxobutan-1-yl]piperidin-4-yl}-1H-indol;
5-Fluor-3-{1-[3-(3,4-dihydro-2H-chinolin-1-yl)-3-oxopropan-1-yl]piperidin-4-yl}-1H-indol;
5-Fluor-3-{1-[4-(3,4-dihydro-2H-chinolin-1-yl)-4-oxobutan-1-yl]piperidin-4-yl}-1H-indol;
5-Fluor-3-{1-[5-(3,4-dihydro-2H-chinolin-1-yl)-5-oxopentan-1-yl]piperidin-4-yl}-1H-indol;
5-Fluor-3-{1-[6-(3,4-dihydro-2H-chinolin-1-yl)-6-oxohexan-1-yl]piperidin-4-yl}-1H-indol;
6-Chlor-3-{1-[3-(3,4-dihydro-2H-chinolin-1-yl)-3-oxopropan-1-yl]piperidin-4-yl]-1H-indol;
6-Chlor-3-{1-[6-(3,4-dihydro-2H-chinolin-1-yl)-6-oxohexan-1-yl]piperidin-4-yl}-1H-indol;
7-Chlor-3-{1-[4-(3,4-dihydro-2H-chinolin-1-yl)-4-oxobutan-1-yl]piperidin-4-yl}-1H-indol;
7-Chlor-3-{1-[5-(3,4-dihydro-2H-chinolin-1-yl)-5-oxopentan-1-yl]piperidin-4-y1}-1H-indol; und
7-Chlor-3-{1-[6-(3,4-dihydro-2H-chinolin-1-yl)-6-oxohexan-1-yl]piperidin-4-yl}-1H-indol;
oder pharmazeutisch akzeptable Salze davon.

14. Pharmazeutische Zusammensetzung, die **dadurch gekennzeichnet ist, dass** sie eine Verbindung gemäss einem der Ansprüche 1 bis 13 in einer therapeutisch wirksamen Menge zusammen mit einem oder mehreren pharmazeutisch akzeptablen Trägern oder Verdünnungsmitteln umfasst.

15. Verwendung einer Verbindung gemäss einem der Ansprüche 1 bis 13 zur Herstellung eines Medikaments, das nützlich ist in der Behandlung von positiven und negativen Symptomen von Schizophrenie, anderen Psychosen, Angststörungen, wie generalisierte Angststörung, Panikstörung und obsessive Zwangsstörung, Depression, Aggression, durch herkömmliche Antipsychotika induzierten Nebenwirkungen, Migräne, kognitiven Störungen, ADHD und in der Verbesserung von Schlaf.

## Revendications

1. Dérivé d'indole substitué de formule I 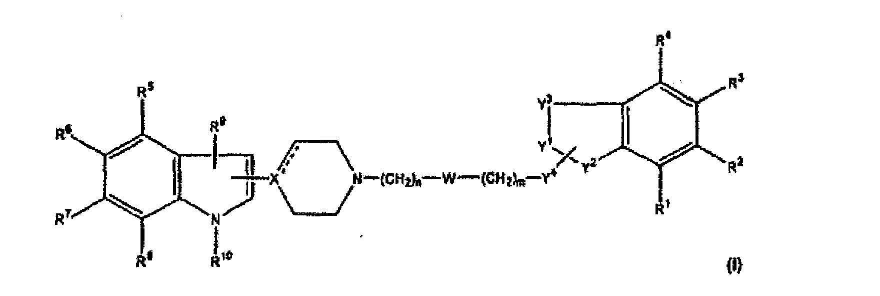 dans laquelle un parmi Y¹ et Y² est N, lequel est lié à Y⁴, et l'autre parmi Y¹ et Y² est CH₂ et Y⁴ est CO, CS, SO ou SO₂; ou
Y³ est Z-CH₂, CH₂-Z ou CH₂CH₂, et Z est O ou S; à la condition que quand Y¹ est N,
Y³ ne soit pas Z-CH₂;
W est une liaison ou n O, S, CO, CS, SO ou groupe SO₂;
n est 0 à 5, m est 0 à 5 et m+n est 1 à 10 ; à la condition que quand W est O ou S, alors n ≥2 et m ≥1; quand W est CO, CS, SO ou SO₂, alors n ≥1 et m ≥1;
X est C, CH ou N ; à la condition que quand X est C, la ligne en pointillé indique une liaison, et quand X est N ou CH, la ligne en pointillé ne soit pas une liaison ;
R¹ à R⁹ sont indépendamment choisis parmi un hydrogène, un halogène, un cyano, un nitro, un amino, un hydroxy, un (alkyle en C₁ à C₆)-amino, un di(alkyle en C₁ à C₆)-amino, un alkyle en C₁ à C₆, un alcényle en C₂ à C₆, un alcynyle en C₂ à C₆, un alcoxy en C₁ à C₆, un alkylthio en C₁ à C₆, un alkyle en C₁ à C₆ substitué avec un hydroxy ou un thiol, un cycloalkyle en C₃ à C₈, un (cycloalkyle en C₃ à C₈)-alkyle en C₁ à C₆, un acyle, un thioacyle, un aryle, un trifluorométhyle, un trifluorométhylsulfonyle et un (alkyle en C₁ à C₆)-sulfonyle ;
R¹⁰ est un hydrogène, un alkyle en C₁ à C₆, un alcényle en C₂ à C₆, un alcynyle en C₂ à C₆, un alkyle en C₁ à C₆ substitué avec un hydroxy ou un thiol, un cycloalkyle en C₃ à C₈, un (cycloalkyle en C₃ à C₈)-alkyle en C₁ à C₆, un aryle, un aryl-(alkyle en C₁ à C₆), un acyle, un thioacyle, un (alkyle en C₁ à C₆)-sulfonyle, un trifluorométhylsulfonyle ou un arylsulfonyle, ou un sel d'addition à un acide pharmaceutiquement acceptable de celui-ci.

2. Composé selon la revendication 1, dans lequel l'indole est lié à X par l'intermédiaire de la position 3 de l'indole.

3. Composé selon les revendications 1 à 2, dans lequel un parmi Y¹ et Y² est N, lequel est lié à Y⁴, et l'autre parmi Y¹ et Y² est CH₂ et Y⁴ est CO.

4. Composé selon la revendication 3, dans lequel Y¹ est un azote lié à Y⁴, Y² est CH₂ et Y⁴ est CO.

5. Composé selon la revendication 3, dans lequel Y² est un atome d'azote lié à Y⁴, Y¹ est CH₂ et Y⁴ est CO.

6. Composé selon les revendications 1 à 5, dans lequel Y³ est CH₂CH₂ ou CH₂Z.

7. Composé selon les revendications 1 à 6, dans lequel X est C.

8. Composé selon les revendications 1 à 6, dans lequel X est N.

9. Composé selon les revendications 1 à 6, dans lequel X est CH.

10. Composé selon les revendications 1 à 9, dans lequel R¹ à R⁹ sont indépendamment choisis parmi un hydrogène, un halogène, un cyano, un nitro, un amino, un (alkyle en C₁ à C₆)-amino, un di(alkyle en C₁ à C₆)-amino, un alkyle en C₁ à C₆, un cycloalkyle en C₃ à C₈ et un trifluorométhyle, et R¹⁰ est un hydrogène, un alkyle en C₁ à C₆ ou un acyle, ou un sel d'addition à un acide pharmaceutiquement acceptable de celui-ci.

11. Composé selon les revendications 1 à 10, dans lequel W est une liaison et n+m est 1 à 6.

12. Composé selon la revendication 11, dans lequel n+m est 3 à 6.

13. Composé selon la revendication 1, qui est choisi parmi :
le 5-fluoro-3-{1-[3-(3,4-dihydro-1H-isoquinolin-2-yl)-3-oxopropan-1-yl]pipéridin-4-yl}-1H-indole;
le 7-chloro-3-{1-[3-(3,4-dihydro-1H-isoquinolin-2-yl)-3-oxopropan-1-yl]pipéridin-4-yl}-1H-indole;
le 5-chloro-3-{1-[4-(3,4-dihydro-2H-quinolin-1-yl)-4-oxobutan-1-yl]pipéridin-4-yl}-1H-indole ;
le 5-fluoro-3-{1-[4-(3,4-dihydro-1H-isoquinolin-2-yl)-4-oxobutan-1-yl]pipéridin-4-yl}-1H-indole;
le 5-chloro-3-{1-[4-(3,4-dihydro-1H-isoquinolin-2-yl)-4-oxobutan-1-yl]pipéridin-4-yl}-1H-indole;
le 4-fluoro-3-{1-[3-(3,4-dihydro-1H-isoquinolin-2-yl)-3-oxopropan-1-yl]pipéridin-4-yl}-1H-indole;
le 4-fluoro-3-{1-[4-(3,4-dihydro-1H-isoquinolin-2-yl)-4-oxobutan-1-yl]pipéridin-4-yl}-1H-indole;
le 4-fluoro-3-{1-[6-(3,4-dihydro-1H-isoquinolin-2-yl)-6-oxohexan-1-yl]pipéridin-4-yl}-1H-indole;
le 4-chloro-3-{1-[4-(3,4-dihydro-1H-isoquinolin-2-yl)-4-oxobutan-1-yl]pipéridin-4-yl}-1H-indole;
le 4-chloro-3-{1-[5-(3,4-dihydro-1H-isoquinolin-2-yl)-5-oxopcntan-1-yl]pipéridin-4-yl}-1H-indole ;
le 4-chloro-3-{1-[6-(3,4-dihydro-1H-isoquinolin-2-yl)-6-oxohexan-1-yl]pipéridin-4-yl}-1H-indole ;
le 5-fluoro-3- {1-[5-(3,4-dihydro-1H-isoquinolin-2-yl)-5-oxopentan-1-yl]pipéridin-4-yl}-1H-indole ;
le 5-fluoro-3-{1-[6-(3,4-dihydro-1H-isoquinolin-2-yl)-6-oxohcxan-1-yl]pipéridin-4-yl}-1H-indole ;
le 6-chloro-3- {1-[3-(3,4-dihydro-1H-isoquinolin-2-yl)-3-oxopropan-1-yl]pipéfidin-4-yl}-1H-indole ;
le 6-chloro-3-{1-[6-(3,4-dihydro-1H-isoquinolin-2-yl)-6-oxohexan-1-yl]pipéridin-4-yl}-1H-indole;
le 7-chloro-3-{1-[4-(3,4-dihydro-1H-isoquinolin-2-yl)-4-oxobutan-1-yl]pipéridin-4-yl}-1H-indole;
le 7-chloro-3-{1-[5-(3,4-dihydro-1H-isoquinolin-2-yl)-5-oxopentan-1-yl]pipéridin-4-yl}-1H-indole ;
le 7-chloro-3-{1-[6-(3,4-dihydro-1H-isoquinolin-2-yl)-6-oxohexan-1-yl]pipéridin-4-yl}-1H-indole ;
le 4-fluoro-3-{1-[3-(3,4-dihydro-2H-quinolin-1-yl)-3-oxopropan-1-yl]pipéridin-4-y1}-1H-indole ;
le 4-fluoro-3-{1-[4-(3,4-dihydro-2H-quinolin-1-yl)-4-oxobutan-1-yl]pipéridin-4-yl}-1H-indole ;
le 4-chloro-3-{1-[3-(3,4-dihydro-2H-quinolin-1-yl)-3-oxopropan-1-yl]pipéridin-4-yl}-1H-indole ;
le 4-chloro-3-{1-[4-(3,4-dihydro-2H-quinolin-1-yl)-4-oxobutan-1-yl]pipéridin-4-yl}-1H-indole;
le 5-fluoro-3-{1-[3-(3,4-dihydro-2H-quinolin-1-yl)-3-oxopropan-1-yl]pipéridin-4-yl}-1H-indole;
le 5-fluoro-3-{1-[4-(3,4-dihydro-2H-quinolin-1-yl)-4-oxobutan-1-yl]pipéridin-4-yl}-1H-indole;
le 5-fluoro-3-{1-[5-(3,4-dihydro-2H-quinolin-1-yl)-5-oxopentan-1-yl]pipéridin-4-yl}-1H-indole;
le 5-fluoro-3-{1-[6-(3,4-dihydro-2H-quinolin-1-yl)-6-oxohexan-1-yl]pipéridin-4-yl}-1H-indole;
le 6-chloro-3-{1-[3-(3,4-dihydro-2H-quinolin-1-yl)-3-oxopropan-1-yl]pipéridin-4-yl}-1H-indole ;
le 6-chloro-3-{1-[6-(3,4-dihydro-2H-quinolin-1-yl)-6-oxohexan-1-yl]pipéridin-4-yl}-1H-indole;
le 7-chloro-3-{1-[4-(3,4-dihydro-2H-quinolin-1-yl)-4-oxobutan-1-yl]pipéridin-4-yl}-1H-indole ;
le 7-chloro-3-{1-[5-(3,4-dihydro-2H-quinolin-1-yl)-5-oxopentan-1-yl]pipéridin-4-yl}-1H-indole ; et
le 7-chloro-3-{1-[6-(3,4-dihydro-2H-quinolin-1-yl)-6-oxohexan-1-yl]pipéridin-4-yl}-1H-indole ;
ou leurs sels pharmaceutiquement acceptables.

14. Composition pharmaceutique **caractérisée en ce qu'**elle comprend un composé selon l'une quelconque des revendications 1 à 13 en une quantité efficace d'un point de vue thérapeutique avec un ou plusieurs supports ou diluants pharmaceutiquement acceptables.

15. Utilisation d'un composé selon l'une quelconque des revendications 1 à 13 pour la fabrication d'un médicament utile dans le traitement des symptômes positifs et négatifs de la schizophrénie, d'autres psychoses, des troubles anxieux, comme un trouble anxieux généralisé, un trouble panique et un trouble obsessionnel-compulsif, la dépression, l'agressivité, les effets secondaires induits par des agents antipsychotiques traditionnels, la migraine, les troubles cognitifs, le TDAH et dans l'amélioration du sommeil.
